# EUROPEAN PATENT APPLICATION

(11) **EP 4 317 191 A1**
(43) Date of publication of application: **07.02.2024**
(21) Application number: 22775734.1
(22) Date of filing: 24.03.2022
(51) Int. Cl.: C07K 19/00, C12P 11/00, C12P 13/02, C12N 15/52, C12N 15/54, C12N 15/62, C12N 9/00, C12N 9/10, C12N 9/12, C12P 21/02

(54) **MODIFIED NICOTINAMIDE PHOSPHORIBOSYLTRANSFERASE**

(30) Priority: 26.03.2021 JP 2021052555
(71) Applicant: Mitsubishi Chemical Corporation, Chiyoda-ku Tokyo 100-8251 (JP)
(72) Inventor: HAYASHI Takahiro, Tokyo 100-8251 (JP); AKIYAMA Takanori, Tokyo 100-8251 (JP)
(74) Representative: Hoffmann Eitle
(86) International application number: PCT/JP2022/013776
(87) International publication number: WO 2022/202952

(57) **Abstract**

The present invention relates to a modified (mutant) nicotinamide phosphoribosyltransferase, and a method for producing nicotinamide mononucleotide using the same. The modified nicotinamide phosphoribosyltransferase (Nampt) of the present invention comprises the amino acid sequence represented by the following SEQ ID NO: 1 and/or the amino acid sequence represented by the following SEQ ID NO: 2, and has improved activity as compared with wild-type Nampt: (a) SEQ ID NO: 1: S-[V/I]-P-A-X₁-X₂-H-S-[T/V/I]-[M/V/I]-X₃, and (b) SEQ ID NO: 2: X₄-[S/I]-D-X₅ wherein X₁ to X₅ are as defined herein.

## Description

### [Technical Field]

### Related Application

The present specification encompasses the contents described in the specification of Japanese Patent Application No. 2021-52555 (filed on March 26, 2021) on which the priority of the present application is based.

### Technical Field:

The present invention relates to a modified (variant) nicotinamide phosphoribosyltransferase, and a method for producing nicotinamide mononucleotide using the same.

### [Background Art]

Nicotinamide mononucleotide (NMN) is an intermediate for the synthesis of nicotinamide adenine dinucleotide (NAD⁺). In recent years, NMN has been found to control the activity of the longevity gene "sirtuin" through conversion to NAD+, and to exhibit an antiaging effect when NMN is administered to mice. It has also been reported that NMN is also effective for the prevention of diseases such as diabetes mellitus, Alzheimer's disease, or heart failure or improvement in their symptoms. As such, use of NMN as a component for functional foods, medicaments, cosmetics, etc. is expected, and research and development on efficient production methods are ongoing with the aim of improvement in productivity.

Methods based on organic synthesis as well as biological methods such as fermentation method and enzymatic method are known as methods for producing NMN. The production of NMN by the enzymatic method is generally composed of: main reaction consisting of three stages of enzymatic reactions, (1) the first reaction of forming ribose-5-phosphate (R5P) from ribose, (2) the second reaction of forming phosphoribosyl pyrophosphate (PRPP) from R5P, and (3) the third reaction of forming NMN from PRPP and nicotinamide (NAM); and support reaction involving ATP regeneration and pyrophosphate degradation.

Nicotinamide phosphoribosyltransferase (Nampt) is a rate-limiting enzyme in a mammalian NAD+ synthesis system and is an enzyme that catalyzes the third reaction of the production route (Non Patent Literature 1). The activity of wild-type Nampt is relatively lower than that of other enzymes and is not sufficient for the industrial production of NMN. Hence, its improvement has been desired. For example, an attempt to improve the production efficiency of NMN has been made by search for Nampt having high activity (HdNampt) (Non Patent Literatures 2 and 3), Nampt activation using a small-molecule compound (Non Patent Literature 4), and improvement in intracellularly accumulated NMN concentration by NMN-differentiation pathway knockdown (Non Patent Literature 5).

The inventors have reported search for highly active Nampt, and a method for improving the production efficiency of NMN by enhancing the expression of phosphoribosyl pyrophosphate synthetase (Prs) and polyphosphate kinase (Ppk) which catalyze the first and second reactions, in addition to Nampt (Patent Literature 1) .

Meanwhile, improvement in the production efficiency of NMN using mutant Nampt obtained by the mutagenesis of the sequence of wild-type Nampt has also been reported (Patent Literature 2). However, in this report, the activity of the resulting mutant Nampt is lower than that of wild-type highly active Nampt used by the present inventors because the wild-type Nampt used therein has very low activity.

### [Citation List]

### [Patent Literature]

[Patent Literature 1] WO2019/065876
[Patent Literature 2] WO2020/129997

### [Non Patent Literature]

[Non Patent Literature 1] Garten et al., "Physiological and pathophysiological roles of NAMPT and NAD metabolism" Nature Reviews Endocrinology volume 11, pp. 535-546 (2015)
[Non Patent Literature 2] Marinescu et al., "β-nicotinamide mononucleotide (NMN) production in Escherichia coli" Scientific Reports volume 8, Article number: 12278 (2018)
[Non Patent Literature 3] Shoji et al., "Metabolic design for selective production of nicotinamide mononucleotide from glucose and nicotinamide" Metab Eng. 2020 Nov 18; S1096-7176 (20) 30176-2
[Non Patent Literature 4] Gardell et al., "Boosting NAD+ with a small molecule that activates NAMPT" Nat Commun. 2019 Jul 19; 10 (1): 3241
[Non Patent Literature 5] Black et al., "Engineering a nicotinamide mononucleotide redox cofactor system for biocatalysis" Nat Chem Biol., 2020 Jan; 16 (1): 87-94

### [Summary of Invention]

### [Technical Problem]

An object of the present invention is to provide nicotinamide phosphoribosyltransferase (Nampt) having high activity and to improve the production efficiency of NMN using the same.

### [Solution to Problem]

The inventors have successfully prepared a modified (mutant) Nampt having high enzymatic activity by preparing a saturation mutant library for each individual residue near the active center of wild-type highly active Nampt, and carrying out activity screening.

Specifically, the present invention provides the following [1] to [15].
[1] A modified nicotinamide phosphoribosyltransferase (Nampt), comprising the amino acid sequence represented by the following SEQ ID NO: 1 and/or the amino acid sequence represented by the following SEQ ID NO: 2,
   the modified Nampt having improved activity as compared with wild-type Nampt:
      (a) SEQ ID NO: 1: S-[V/I]-P-A-X₁-X₂-H-S-[T/V/I]-[M/V/I]-X₃, and
      (b) SEQ ID NO: 2: X₄-[S/I]-D-X₅
   wherein at least one of X₁ to X₃ is different from the corresponding amino acid of the wild type; X₂ represents an amino acid other than A and Q; X₃ represents an amino acid other than M; and the parentheses [ ] represent any one of the amino acids within the parentheses [ ].
[2] A modified Nampt, comprising the amino acid sequence represented by the following SEQ ID NO: 1 and/or the amino acid sequence represented by the following SEQ ID NO: 2,
   the modified Nampt having improved activity as compared with wild-type Nampt:
      (a) SEQ ID NO: 1: S-[V/I]-P-A-X₁-X₂-H-S-[T/V/I]-[M/V/I]-X₃, and
      (b) SEQ ID NO: 2: X₄-[S/I]-D-X₅
   wherein at least one of X₁ to X₅ is different from the corresponding amino acid of the wild type; X₁ represents any amino acid selected from neutral amino acids and aliphatic amino acids; X₂ represents any amino acid selected from aliphatic amino acids, acidic amino acids and neutral hydrophilic amino acids; X₃ represents any amino acid selected from nonpolar amino acids; X₄ represents any amino acid selected from aliphatic amino acids; X₅ represents any amino acid selected from amino acids other than aromatic amino acids; and the parentheses [ ] represent any one of the amino acids within the parentheses [ ].
[3] A modified nicotinamide phosphoribosyltransferase (Nampt), comprising the amino acid sequence represented by the following SEQ ID NO: 1 and/or the amino acid sequence represented by the following SEQ ID NO: 2,
   the modified Nampt having improved activity as compared with wild-type Nampt:
      (a) SEQ ID NO: 1: S-[V/I]-P-A-X₁-X₂-H-S-[T/V/I]-[M/V/I]-X₃, and
      (b) SEQ ID NO: 2: X₄-[S/I]-D-X₅
   wherein at least one of X₁, X₄, and X₅ is different from the corresponding amino acid of the wild type; X₁ represents any amino acid selected from neutral amino acids and aliphatic amino acids; X₄ represents any amino acid selected from aliphatic amino acids; X₅ represents any amino acid selected from amino acids other than aromatic amino acids; X₂ and X₃ each represent any amino acid; and the parentheses [ ] represent any one of the amino acids within the parentheses [ ].
[4] The modified Nampt according to any of [1] to [3], wherein
   when the amino acid sequence of the modified Nampt is aligned with the amino acid sequence represented by SEQ ID NO: 3,
   the amino acid sequence represented by SEQ ID NO: 1 is located at positions corresponding to positions 243 to 253 of SEQ ID NO: 3, and
   the amino acid sequence represented by SEQ ID NO: 2 is located at positions corresponding to positions 278 to 281 of SEQ ID NO: 3.
[5] The modified Nampt according to any of [1] to [4], wherein the modified Nampt has one or two or more substitutions selected from the following 1) to 5):
   1) a substitution of the wild-type amino acid at X₁ by T,
   2) a substitution of the wild-type amino acid at X₂ by G,
   3) a substitution of the wild-type amino acid at X₃ by G or A,
   4) a substitution of the wild-type amino acid at X₄ by A, and
   5) a substitution of the wild-type amino acid at X₅ by S, A, N, or I.
[6] The modified Nampt according to any of [1] to [5], wherein the wild-type sequence of the modified Nampt has the following amino acid sequence (1) or (2):
   (1) the amino acid sequence represented by any of SEQ ID NOs: 3, 5, 7, 9, 11, 13, 15, 17, 19, 21, and 23, and
   (2) an amino acid sequence which has 90% or higher identity to the amino acid sequence represented by any of SEQ ID NOs: 3, 5, 7, 9, 11, 13, 15, 17, 19, 21, and 23 and encodes a protein having Nampt activity.
[7] The modified Nampt according to any of [1] to [6], wherein the modified Nampt is derived from the genus *Haemophilus,* the genus *Meiothermus,* the genus *Xanthomonas,* the genus *Sphingopyxis,* the genus *Sphingopyxis,* the genus *Chitinophaga,* the genus *Burkholderia,* the genus *Pedobacter,* the genus *Microbulbifer,* the genus *Labrenzia,* or the genus *Luteibacter.*
[8] A method for producing nicotinamide mononucleotide by contacting phosphoribosyl pyrophosphate and nicotinamide with each other in the presence of a modified Nampt according to any of [1] to [7].
[9] The method according to [8], wherein the phosphoribosyl pyrophosphate is produced from ribose-5-phosphate in the presence of phosphoribosyl pyrophosphate synthetase.
[10] The method according to [9], wherein the ribose-5-phosphate is produced from ribose in the presence of ribokinase.
[11] DNA encoding a modified nicotinamide phosphoribosyltransferase (Nampt) according to any of [1] to [7].
[12] A recombinant vector comprising DNA according to [11] .
[13] A transformant comprising a recombinant vector according to [12].
[14] A method for producing Nampt, comprising culturing a transformant according to [13], and collecting nicotinamide phosphoribosyltransferase (Nampt) from the resulting cultures.
[15] A method for producing nicotinamide mononucleotide, comprising contacting phosphoribosyl pyrophosphate and nicotinamide with each other in the presence of a modified Nampt according to any of [1] to [7] or cultures obtained by culturing a transformant according to [9] or a treated product of the cultures.

### [Advantageous Effects of Invention]

The Nampt of the present invention improves the production efficiency of NMN and also allows reduction of starting material cost.

### [Brief Description of Drawing]

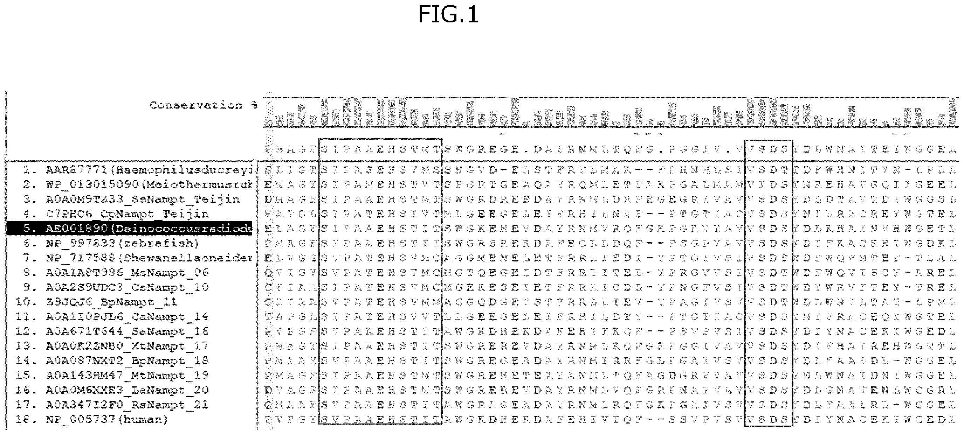

[Figure 1] Figure 1 shows the alignment of the amino acid sequences of Nampt derived from various microbes.

### [Description of Embodiments]

Abbreviations used herein are each as defined below.
Nampt: Nicotinamide phosphoribosyltransferase
Prs: Phosphoribosyl pyrophosphate synthetase
Rbk: Ribokinase
Ppk: Polyphosphate kinase
PPase: Pyrophosphatase
NMN: Nicotinamide mononucleotide
PRPP: Phosphoribosyl pyrophosphate
NAM: Nicotinamide
R5P: Ribose-5-phosphate
NR: Nicotinamide riboside
NaMN: Nicotinic acid mononucleotide
NAD: Nicotinamide adenine dinucleotide
PPi: Pyrophosphate
PolyP: Polyphosphate

### 1. Nicotinamide phosphoribosyltransferase (Nampt)

### 1.1 Wild-type Nampt

Nampt (EC number: 2.4.2.12) is generally known to participate in the NAD (nicotinamide adenine dinucleotide) salvage pathway. In the present invention, Nampt is an enzyme that is used for the reaction of forming NMN from PRPP and NAM (third reaction). For the NMN synthesis reaction from PRPP and NAM using Nampt, ATP is not essential under ordinary circumstances. However, it is reported that Nampt has ATP hydrolytic activity, and through the hydrolysis of ATP, Nampt is autophosphorylated so that enzymatic parameters or chemical equilibrium is shifted in favor of NMN formation (Biochemistry 2008, 47, 11086-11096).

Examples of the Nampt include human (*Homo sapiens*)-derived Nampt (NP_005737), mouse (*Mus musculus*)*-*derived Nampt (NP_067499), rat (*Rattus norvegicus*)-derived Nampt (NP_808789), zebrafish (*Danio rerio*)-derived Nampt (NP_997833), and Nampt derived from bacteria such as *Haemophilus ducreyi* (AAR87771), *Deinococcus radiodurans* (AE001890), *Oenococcus oeni* (KZD13878), and *Shewanella oneidensis* (NP_717588). Among them, bacterium-derived Nampt is preferred from the viewpoint of enzymatic activity in NMS synthesis. In this context, the bacteria are a group of prokaryotes having no nuclear membrane and are an organism group including *Escherichia coli, Bacillus subtilis,* and cyanobacteria.

Specifically, the Nampt is preferably derived from the genus *Haemophilus,* the genus *Meiothermus,* the genus *Xanthomonas,* the genus *Sphingopyxis,* the genus *Sphingopyxis,* the genus *Chitinophaga,* the genus *Burkholderia,* the genus *Pedobacter,* the genus *Microbulbifer,* or the genus *Labrenzia.*

Table 1 shows the origin, GenBank accession No., and SEQ ID NO in the sequence listing of the Nampt that may be used in the present invention. Figure 1 shows the alignment of the amino acid sequence of each Nampt.

**[Table 1]**

| Origin | Protein ID | Amino acid sequence | Nucleotide sequence |
|---|---|---|---|
| Haemophilus ducreyi | AAR87771 | SEQ ID NO:3 | SEQ ID NO:4 |
| Meiothermus ruber | A0A0S7ALY5 | SEQ ID NO:5 | SEQ ID NO:6 |
| Xanthomonas translucens | A0A0K2ZNB0 | SEQ ID NO:7 | SEQ ID NO:8 |
| Sphingopyxis sp. C-1 | A0A0M9TZ33 | SEQ ID NO:9 | SEQ ID NO:10 |
| Chitinophaga pinensis | C7PHC6 | SEQ ID NO:11 | SEQ ID NO:12 |
| Chitinophaga arvensicola | A0A1I0PJL6 | SEQ ID NO:13 | SEQ ID NO:14 |
| Burkholderia pyrrocinia | A0A087NXT2 | SEQ ID NO:15 | SEQ ID NO:16 |
| Pedobacter alluvionis | A0A497YC34 | SEQ ID NO:17 | SEQ ID NO:18 |
| Microbulbifer thermotolerans | AOA143HM47 | SEQ ID NO:19 | SEQ ID NO:20 |
| Labrenzia aggregata | A0A0M6XXE3 | SEQ ID NO:21 | SEQ ID NO:22 |
| Luteibacter sp. | AOA1K1R8E3 | SEQ ID NO:23 | SEQ ID NO:24 |

The Nampt according to the present invention is not limited to those having the sequences described above. The Nampt of the present invention also includes a protein which comprises an amino acid sequence having approximately 60% or higher, preferably approximately 70% or higher, more preferably approximately 80% or higher, further preferably approximately 90% or higher, particularly preferably approximately 95% or higher, most preferably approximately 98% or higher homology or identity to the amino acid sequence as set forth in any of SEQ ID NOs: 3, 5, 7, 9, 11, 13, 15, 17, 19, 21, and 23 and has Nampt activity.

The Nampt of the present invention also includes a protein which comprises an amino acid sequence derived from the amino acid sequence as set forth in any of SEQ ID NOs: 3, 5, 7, 9, 11, 13, 15, 17, 19, 21, and 23 by the deletion, substitution or addition of one or several, specifically, 1 to 20, preferably 1 to 10, more preferably 1 to 5, further preferably 1 or 2 amino acids and has Nampt activity.

The gene of the Nampt according to the present invention encodes any of the amino acid sequences described above. As one example, the Nampt gene has the nucleotide sequence as set forth in any of SEQ ID NOs: 4, 6, 8, 10, 12, 14, 16, 18, 20, 22, and 24. However, the Nampt gene of the present invention also includes a gene which comprises a nucleotide sequence having approximately 60% or higher, preferably approximately 70% or higher, more preferably approximately 80% or higher, further preferably approximately 90% or higher, particularly preferably approximately 95% or higher, most preferably approximately 98% or higher identity to the nucleotide sequence and comprises a nucleotide sequence encoding a protein having Nampt activity.

The Nampt gene of the present invention also includes a gene comprising a nucleotide sequence which hybridizes under stringent conditions to the nucleotide sequence as set forth in any of SEQ ID NOs: 4, 6, 8, 10, 12, 14, 16, 18, 20, 22, and 24 and encodes a protein having Nampt activity. Examples of the stringent conditions can include, but are not limited to, conditions under which hybridization is performed by incubating a DNA-immobilized nylon membrane together with a probe at 65°C for 20 hours in a solution containing 6 × SSC (1 × SSC refers to 8.76 g of sodium chloride and 4.41 g of sodium citrate dissolved in 1 L of water), 1% SDS, 100 µg/ml salmon sperm DNA, 0.1% bovine serum albumin, 0.1% polyvinylpyrrolidone, and 0.1% Ficoll. Those skilled in the art can set the hybridization conditions by taking into consideration such conditions such as the salt concentration of the buffer and the temperature as well as other conditions such as a probe concentration, a probe length, and a reaction time. Examples of the washing conditions following hybridization can include "2 × SSC, 0.1% SDS, 42°C", "1 × SSC, 0.1% SDS, 37°C", and more stringent conditions such as "1 × SSC, 0.1% SDS, 65°C" and "0.5 × SSC, 0.1% SDS, 50°C".

### 1.2 Modified Nampt

The modified Nampt of the present invention is a novel modified Nampt that contains a mutation of at least one amino acid residue and has more improved activity than wild-type activity.

The origin of the modified Nampt of the present invention is not particularly limited. For example, Nampt registered in the GenBank database (http://www.ncbi.nlm.nih.gov/entrez/query.fcgi?CMD=search &DB=protein) provided by the National Center for Biotechnology Information (NCBI), or Nampt described in a literature known in the art can be used. Specifically, Nampt known in the art, described in the section 1.1, can be used.

The modified Nampt of the present invention comprises the amino acid sequence represented by the following SEQ ID NO: 1 and/or the amino acid sequence represented by the following SEQ ID NO: 2:
(a) SEQ ID NO: 1: S-[V/I]-P-A-X₁-X₂-H-S-[T/V/I]-[M/V/I]-X₃, and
(b) SEQ ID NO: 2: X₄-[S/I]-D-X₅
wherein at least one of X₁ to X₃ is different from the corresponding amino acid of the wild type, and the parentheses [ ] represent any one of the amino acids within the parentheses [ ].

In the first embodiment, at least one of X₁ to X₅ is different from the corresponding amino acid of the wild type; X₂ represents an amino acid other than A and Q; and X₃ represents an amino acid other than M.

In the second embodiment, at least one of X₁ to X₅ is different from the corresponding amino acid of the wild type; X₁ represents any amino acid selected from neutral amino acids and aliphatic amino acids; X₂ represents any amino acid selected from aliphatic amino acids, acidic amino acids and neutral hydrophilic amino acids; X₃ represents any amino acid selected from nonpolar amino acids; X₄ represents any amino acid selected from aliphatic amino acids; and X₅ represents any amino acid selected from amino acids other than aromatic amino acids.

In the third embodiment, at least one of X₁, X₄, and X₅ is different from the corresponding amino acid of the wild type; X₁ represents any amino acid selected from neutral amino acids and aliphatic amino acids; X₄ represents any amino acid selected from aliphatic amino acids; and X₅ represents any amino acid selected from amino acids other than aromatic amino acids. X₂ and X₃ each represent any amino acid.

In this context, the "acidic amino acid" is, for example, aspartic acid (D) or glutamic acid (E), preferably glutamic acid (E).

The "neutral amino acid" is, for example, glycine (G), alanine (A), phenylalanine (F), leucine (L), isoleucine (I), cysteine (C), methionine (M), tyrosine (Y), valine (V), threonine (T), serine (S), proline (P), tryptophan (W), asparagine (N), or glutamine (Q), preferably valine (V), threonine (T), alanine (A), glycine (G), leucine (L), serine (S), asparagine (N), or isoleucine (I), more preferably threonine (T), alanine (A), glycine (G), leucine (L), serine (S), asparagine (N), or isoleucine (I).

The "aliphatic amino acid" is, for example, alanine (A), leucine (L), isoleucine (I), valine (V), or phenylalanine (F), preferably alanine (A), leucine (L), isoleucine (I), or valine (V), more preferably alanine (A) or isoleucine (I).

The "hydrophilic amino acid" is, for example, aspartic acid (D), glutamic acid (E), lysine (K), arginine (R), threonine (T), serine (S), asparagine (N), glutamine (Q), or histidine (H), preferably threonine (T), serine (S), asparagine (N), or glutamine (Q), more preferably threonine (T) or serine (S).

The "nonpolar amino acid" is, for example, alanine (A), valine (V), leucine (L), isoleucine (I), methionine (M), phenylalanine (F), tryptophan (W), proline (P), glycine (G), or cysteine (C), preferably alanine (A), valine (V), leucine (L), isoleucine (I), or glycine (G), more preferably alanine (A), valine (V), or glycine (G).

The "aromatic amino acid" is, for example, phenylalanine (F), tryptophan (W), or tyrosine (Y), preferably phenylalanine (F) or tryptophan (W), more preferably tryptophan (W).

The sequences represented by SEQ ID NOs: 1 and 2 are motif sequences commonly located in the amino acid sequences of Nampt known in the art. As shown in Figure 1, when the amino acid sequence of the modified Nampt is aligned with the *Haemophilus ducreyi*-derived (AAR87771) amino acid sequence represented by SEQ ID NO: 3,
(a) the amino acid sequence represented by SEQ ID NO: 1 is located at positions corresponding to positions 243 to 253 of SEQ ID NO: 3, and
(b) the amino acid sequence represented by SEQ ID NO: 2 is located at positions corresponding to positions 278 to 281 of SEQ ID NO: 3.

At least one of X₁ to X₅ can be different from the corresponding amino acid of the wild type as long as the activity of the Nampt is improved as compared with the wild type.

Preferred substitutions at X₁ to X₅ include the following:
1) X₁: a substitution of the wild-type amino acid by T or V, more preferably by T,
2) X₂: a substitution of the wild-type amino acid by A or G, more preferably by G,
3) X₃: a substitution of the wild-type amino acid by V, G or A, more preferably by G or A,
4) X₄: a substitution of the wild-type amino acid by A or L, more preferably by A, and
5) X₅: a substitution of the wild-type amino acid by G, S, A, N, or I, more preferably by S, A, N, or I.

The modified Nampt of the present invention may have two or more mutations at X₁ to X₅ described above (combined substitutions (mutations)). The introduction of such a plurality of mutations may achieve higher activity than that brought about by a single mutation.

Such combined substitutions include the following combinations:
2 substitutions: X₁ and X₂, X₁ and X₃, X₁ and X₄, X₁ and X₅, X₂ and X₃, X₂ and X₄, X₂ and X₅, X₃ and X₄, X₃ and X₅, and X₄ and X₅,
3 substitutions: X₁, X₂ and X₃, X₁, X₂ and X₄, X₁, X₂ and X₅, X₁, X₃ and X₄, X₁, X₃ and X₅, X₁, X₄ and X₅, X₂, X₃ and X₄, X₂, X₃ and X₅, X₂, X₄ and X₅, and X₃, X₄ and X₅,
4 substitutions: X₁, X₂, X₃ and X₄, X₁, X₂, X₃ and X₅, X₁, X₂, X₄ and X₅, X₁, X₃, X₄ and X₅, and X₂, X₃, X₄ and X₅, and
5 substitutions: X₁, X₂, X₃, X₄ and X₅.

Among them, combined substitutions at X₁ and X₃, X₁ and X₄, or X₁ and X₅ are preferred, and combined substitutions at X₃ and X₅ or X₄ and X₅ are more preferred.

More specifically, a combination of a substitution at X₁ by T and a substitution at X₃ by G, a combination of a substitution at X₁ by T and a substitution at X₄ by A, a combination of a substitution at X₁ by T and a substitution at X₅ by S, a combination of a substitution at X₃ by G and a substitution at X₅ by S, or a combination of a substitution at X₁ by T and a substitution at X₃ by T is preferred, and a combination of a substitution at X₃ by A and a substitution at X₅ by S or a combination of a substitution at X₄ by G and a substitution at X₃ by S is more preferred.

X₁ to X₅ described above correspond to amino acids at positions 247, 248, 253, 278, and 281, respectively, of the amino acid sequence of SEQ ID NO: 3. Thus, the modified nicotinamide phosphoribosyltransferase (Nampt) of the present invention can also be described as a modified Nampt wherein, when its amino acid sequence is aligned with the amino acid sequence of *Haemophilus ducreyi*-derived (AAR87771) Nampt represented by SEQ ID NO: 3, at least one amino acid selected from amino acids at positions respectively corresponding to positions 247, 248, 253, 278, and 281 of SEQ ID NO: 3 is substituted by an amino acid different from the corresponding amino acid of the wild type, and the modified Nampt has improved activity as compared with wild-type Nampt.

In this case, preferred substitutions can be described as follows:
1) a substitution of the wild-type amino acid at a position (X₁) corresponding to position 247 of SEQ ID NO: 3 by T or V, more preferably by T,
2) a substitution of the wild-type amino acid at a position (X₂) corresponding to position 248 of SEQ ID NO: 3 by A or G, more preferably by G,
3) a substitution of the wild-type amino acid at a position (X₃) corresponding to position 253 of SEQ ID NO: 3 by V, G or A, more preferably by G or A,
4) a substitution of the wild-type amino acid at a position (X₄) corresponding to position 278 of SEQ ID NO: 3 by A or L, more preferably by A, and
5) a substitution of the wild-type amino acid at a position (X₅) corresponding to position 281 of SEQ ID NO: 3 by G, S, A, N, or I, more preferably by S, A, N, or I.

The modified Nampt of the present invention has higher activity, preferably at least 1.2 times higher activity, more preferably at least 1.4 times higher activity, than that of the wild type.

In this context, the "activity" means Nampt activity, i.e., enzymatic activity that catalyzes the reaction of forming NMN from PRPP and NAM. In activity measurement, the activity can be calculated by contacting the substrates PRPP and NAM with Nampt to convert these substrates to NMN, followed by the quantification of this NMN.

The reaction conditions involve a substrate concentration in the range of 10 to 100 mM, a reaction temperature in the range of 10°C to 40°C, and a reaction time in the range of 1 hour to 40 hours. The reaction is terminated by the addition of methanol and EDTA (ethylenediaminetetraacetic acid). Then, the formed NMN can be quantified by HPLC (high-performance liquid chromatography) analysis.

The modified Nampt of the present invention is obtained by introducing any of the amino acid substitutions mentioned above to known Nampt, and comparing its enzymatic activity with the wild type for selection. Examples of the mutagenesis method include site-directed mutagenesis and genome editing using site-directed nuclease.

Various methods such as QuikChange method as well as Kunkel method and gapped duplex method are known about the site-directed mutagenesis. Such a method can be conveniently carried out using a commercially available kit for mutagenesis.

A method known in the art such as genome editing using zinc finger nuclease (ZFN), transcription activator-like effector nuclease (TALEN), or CRISPR/Cas9 can be used as the genome editing using site-directed nuclease. Such a method can be carried out using a commercially available kit.

### 2. Gene (DNA) encoding modified Nampt

The present invention also provides a gene (DNA) encoding the modified Nampt of the present invention.

The "DNA encoding the modified Nampt" of the present invention also includes DNA which hybridizes under stringent conditions to DNA having a nucleotide sequence complementary to DNA encoding the modified Nampt and encodes a protein having higher Nampt activity than that of the wild type.

The "stringent conditions" are conditions for washing following hybridization and mean conditions involving a salt concentration of 300 to 2000 mM and a temperature of 40 to 75°C, preferably a salt concentration of 600 to 900 mM and a temperature of 65°C. Examples thereof can include conditions of 2 × SSC and 50°C. Those skilled in the art can appropriately set the conditions for obtaining the DNA encoding the Nampt of the present invention by taking into consideration such conditions such as the salt concentration of the buffer and the temperature as well as other conditions such as a probe concentration, a probe length, and a reaction time.

For detailed procedures of the hybridization method, see, for example, Molecular Cloning, A Laboratory Manual 2nd ed. (Cold Spring Harbor Laboratory Press (1989)). Examples of the hybridizing DNA include DNA comprising a nucleotide sequence having at least 40% or higher, preferably 60%, further preferably 90% or higher sequence identity to the gene DNA of the present invention, and partial fragments thereof.

### 3. Expression vector

The present invention also provides an expression vector comprising the gene (DNA) encoding the modified Nampt of the present invention. As mentioned above, a plurality of enzymes participate in the synthesis of NMN by the enzymatic method. The expression vector of the present invention may comprise, in addition to the gene encoding the modified Nampt, genes (DNA) encoding other enzymes that act in the NMN formation route. The expression vector may comprise, for example, all of genes encoding Nampt as well as Prs and Ppk mentioned later, or may be an expression vector comprising genes encoding Nampt and Prs or an expression vector comprising genes encoding Nampt and Ppk.

The expression vector used in the present invention can be prepared by an approach known in the art. In general, an expression cassette is constructed by inserting a transcriptional promoter to upstream of a gene encoding a predetermined enzyme and, optionally, a terminator to downstream thereof, and this cassette can be inserted to an expression vector. Alternatively, when the expression vector already has a transcriptional promoter and/or a terminator, a gene encoding a predetermined enzyme can be inserted to between the transcriptional promoter and/or the terminator of the vector without constructing an expression cassette. When one expression vector comprises genes encoding two or more enzymes as mentioned above, all of these genes may be inserted downstream of the same promoter or may be inserted downstream of different promoters. The type of the promoter is not particularly limited as long as the promoter permits expression suitable for a host. Examples of the promoter that can be used for an *Escherichia coli* host include T7 promoter, trp promoter, lac promoter, random phage-derived PL promoter and PR promoter, tac promoter, and trc promoter.

The gene encoding each predetermined enzyme may be obtained, for example, by (i) preparing primers according to nucleotide sequence information, followed by amplification with the genome or the like as a template, or by (ii) synthesizing DNA by organic synthesis according to amino acid sequence information on the enzyme. The gene may be optimized according to host cells for transformants.

For example, a method using restriction enzymes or a method using topoisomerase can be used for inserting the gene encoding a predetermined enzyme to the expression vector. If necessary, an appropriate linker may be added for insertion. A ribosomal binding sequence such as a SD sequence or a Kozak sequence is known as a nucleotide sequence important for translation into amino acids. Such a sequence may be inserted upstream of the gene. For insertion, the amino acid sequence encoded by the gene may be partially substituted. The vector preferably comprises a factor for selecting the transformant of interest (selective marker). Examples of the selective marker include drug resistance genes, auxotrophic complementary genes, and assimilation-imparting genes. The selective marker can be selected according to a purpose or a host. Examples of the drug resistance gene for use as a selective marker in *Escherichia coli* include ampicillin resistance gene, kanamycin gene, dihydrofolate reductase gene, and neomycin resistance gene.

A suitable expression vector selected from plasmid DNA, bacteriophage DNA, retrotransposon DNA, artificial chromosomal DNA, and the like may be used according to a host. For example, in the case of using *Escherichia coli* as a host, examples thereof can include pTrc99A (GE Healthcare Biosciences Corp.), pACYC184 (Nippon Gene Co., Ltd.), pMW118 (Nippon Gene Co., Ltd.), and pET series vectors (Novagen). Examples of the vector having two or more insertion sites can include pETDuet-1 (Novagen). If necessary, modified forms of these vectors may be used.

The expression vector can enhance the expression of a predetermined enzyme by inserting an expression cassette containing a suitable promoter, terminator, marker gene, and the like linked to the gene encoding the predetermined enzyme into the genome of a host. A method known in the art can be used as a method for obtaining a transformant with the expression cassette inserted into the genome. For example, in the case of inserting the expression cassette into the genome by homologous recombination, a host can be transformed with a plasmid that has the expression cassette for the predetermined enzyme and the sequence of an arbitrary genomic region and is incapable of replicating in the host, to obtain a transformant with the whole plasmid or the expression cassette inserted into the genome. In this respect, a plasmid carrying a negative selective marker such as SacB gene (which encodes levansucrase) or a plasmid having a temperature-sensitive (ts) replication mechanism may be used to efficiently obtain a transformant having only the expression cassette inserted into the genome through two homologous recombination events. Alternatively, a transformant with the expression cassette inserted at a random position on the genome may be obtained by transformation with a DNA fragment consisting of only the expression cassette.

In the case of using an enzyme gene that a host originally carries on the genome (an endogenous enzyme gene) as an enzyme other than the Nampt, the expression thereof may be enhanced by replacing a promoter of the enzyme gene on the genome with a strong one. Any of the promoters for use in the expression vector mentioned above can also be used as the promoter.

### 4. Transformant

The present invention also provides a transformant comprising the gene (DNA) encoding the modified Nampt of the present invention or the expression vector. The transformant of the present invention may comprise, in addition to the DNA encoding the modified Nampt, DNA encoding any of other enzymes that act in the NMN production route, as described in the preceding section.

The host of the transformant is not particularly limited as long as the cell can express a predetermined enzyme through a protein expression system using the expression vector or the like. Examples thereof include: bacterial such as *Escherichia coli, Bacillus subtilis,* and actinomycete (e.g., the genus *Rhodococcus* and the genus *Corynebacterium*)*;* yeasts (e.g., the genus *Saccharomyces,* the genus *Candida,* and the genus *Pichia*)*;* filamentous fungi; plant cells; and animal cells such as insect cells and mammalian cells. Among them, *Escherichia coli,* a bacterium of the genus *Corynebacterium,* a bacterium of the genus *Rhodococcus,* a yeast of the genus *Saccharomyces,* a yeast of the genus *Candida,* or a yeast of the genus *Pichia* is preferred, and *Escherichia coli* is more preferred.

Examples of the *Escherichia coli* include *Escherichia coli* K12 and B strains, and derivative strains derived from these wild strains, for example, W3110, JM109, XL1-Blue (e.g., XL1-BlueMRF'), K802, C600, BL21, BL21 (DE3), and BN8 (WO2019/065876) strains.

The method for transferring the expression vector to the host is not particularly limited as long as the method is suitable for the host. Examples of the method that may be used include electroporation, a method using calcium ions, spheroplast method, lithium acetate method, calcium phosphate method, and lipofection.

The transformant harboring the expression vector can be cultured by a method suitable for the cell (microbial cell) used as the host to express each predetermined enzyme. As mentioned above, in the case of combining transformants separately comprising respective genes encoding predetermined enzymes, for example, in the case of preparing and combining a transformant expressing Nampt and Prs and a transformant expressing Ppk, these transformants may be cultured in the same medium or may be cultured in separate media and then mixed.

In the case of performing NMN formation reaction using transformants, dormant microbial cells, microbial cells having improved membrane permeability, inactivated microbial cells or homogenized microbial cells prepared from the transformants, cell-free extracts prepared from the homogenized microbial cells, or stabilized products (for details, see matter regarding the second step mentioned later) obtained by the stabilization treatment thereof, NMN may be unable to be efficiently produced due to the degradation or side reaction of a reactant (substrate) ribose or NAM or the product NMN. In this case, a host obtained by the disruption or deletion of a gene causative of degradation or side reaction can be used. Specifically, a host obtained by the deletion or disruption of a gene encoding at least one enzyme classified into any of the following EC Nos. (a) to (i) can be used.
(a) EC 3.1.3.5
(b) EC 3.5.1.19
(c) EC 2.4.2.1
(d) EC 3.5.1.42
(e) EC 1.17.2.1
(f) EC 1.17.1.5
(g) EC 3.2.2.1
(h) EC 3.2.2.3
(i) EC 3.2.2.14

The enzyme classified into (a) EC 3.1.3.5 is 5'-nucleotidase and includes an enzyme that catalyzes the reaction of forming nicotinamide riboside (NR) and phosphate by the hydrolysis of NMN. Examples thereof include *Escherichia coli* UshA, SurE, YrfG, and YjjG. Particularly, UshA or a homolog thereof is preferred.

The enzyme classified into (b) EC 3.5.1.19 is nicotinamidase and includes an enzyme that participates in NAM degradation. Examples thereof include *Escherichia coli* PncA.

The enzyme classified into (c) EC 2.4.2.1 is purine-nucleoside phosphorylase and includes an enzyme that catalyzes the reaction of forming NAM and ribose-1-phosphate (R1P) by the phosphorolysis of NR. Examples thereof include *Escherichia coli* DeoD and homologs thereof.

The enzyme classified into (d) EC 3.5.1.42 is nicotinamide mononucleotide deaminase and includes an enzyme that catalyzes the reaction of forming NaMN and ammonia by the hydrolysis of NMN. Examples thereof include *Escherichia coli* PncC and homologs thereof.

Each of the enzymes classified into (e) EC 1.17.2.1 and (f) EC 1.17.1.5 is nicotinate dehydrogenase and includes an enzyme that is capable of participating in the formation of hydroxynicotinate as a by-product from NAM.

The enzyme classified into (g) EC 3.2.2.1 is purine nucleosidase and includes an enzyme that catalyzes the reaction of forming NAM and ribose by the hydrolysis of NR. Examples thereof include Pu-N and homologs thereof.

The enzyme classified into (h) EC 3.2.2.3 is uridine nucleosidase and includes an enzyme that is capable of catalyzing the reaction of forming NAM and ribose by the hydrolysis of NR. Examples thereof include URH1 and homologs thereof.

The enzyme classified into (i) EC 3.2.2.14 is NMN nucleosidase and includes an enzyme that catalyzes the reaction of forming NAM and R5P by the hydrolysis of NMN. In the present invention, a gene encoding NMN nucleosidase is preferably disrupted or deleted.

The gene to be deleted or disrupted is preferably a gene encoding the enzyme classified into EC No. shown in (d) and a gene encoding at least one enzyme classified into EC No. shown in any of (a), (c), (g), (h) and (i), more preferably a gene encoding the enzyme classified into EC No. shown in (d) and a gene encoding the enzyme classified into EC No. shown in (a).

The method for disrupting or deleting the gene is not particularly limited and can be performed by a gene disruption or deletion method known in the art. Examples thereof include a method using a linearized fragment for gene disruption or deletion, a method using a cyclic gene disruption or deletion plasmid containing no replication origin, a method using group II intron, Red-ET homologous recombination, and a method using ZFN, TALEN, or CRISPR/Cas9 genome editing.

### 5. Method for producing modified Nampt

The modified Nampt can be produced by culturing the transformant, and collecting a protein having Nampt activity from the resulting cultures. The present invention also provides such a method for producing the modified Nampt.

The "cultures" as used herein encompasses all of a culture supernatant, cultured cells, cultured microbial cells, and homogenates of the cells or the microbial cells.

The culture of the transformant is performed in accordance with a method that is usually used in host culture. Any of a natural medium and a synthetic medium may be used as a medium for the culture of the transformant of the present invention as long as the medium contains a carbon source, a nitrogen source, inorganic salts, and the like assimilable by the host microbe and enables the transformant to be efficiently cultured. Examples of the carbon source include hydrocarbons such as glucose, galactose, fructose, sucrose, raffinose and starch, organic acids such as acetic acid and propionic acid, and alcohols such as ethanol and propanol. Examples of the nitrogen source include ammonia, ammonium salts of inorganic acids or organic acids, such as ammonium chloride, ammonium sulfate, ammonium acetate and ammonium phosphate, and other nitrogen-containing compounds.

In addition, peptone, yeast extracts, meat extracts, corn steep liquor, various amino acids, and the like may be used. Examples of the inorganic matter include monobasic potassium phosphate, dibasic potassium phosphate, magnesium phosphate, magnesium sulfate, sodium chloride, ferrous sulfate, manganese sulfate, zinc sulfate, copper sulfate, and calcium carbonate. If necessary, an antifoaming agent may be added to the medium in order to prevent foaming during culture. A compound serving as an inducer for enzyme expression may be further added to the medium.

The culture may be performed under selective pressure in order to prevent the dropout of the vector and the gene of interest during the culture. Specifically, a drug appropriate for a drug resistance gene used as a selective marker may be added to the medium, or a nutritional factor appropriate for an auxotrophic complementary gene used as a selective marker may be removed from the medium.

When the selective marker is an assimilation-imparting gene, a corresponding assimilation factor can be added, if necessary, as a sole factor. For example, in the case of culturing *Escherichia coli* transformed with a vector containing ampicillin resistance gene, ampicillin may be added, if necessary, during culture.

In the case of culturing the transformant obtained by transformation with a recombinant vector using an inducible promoter as a promoter, an inducer may be added, if necessary, to the medium. For example, when the transformant obtained by transformation with an expression vector having a promoter inducible with isopropyl-β-D-thiogalactoside (IPTG) is cultured, IPTG or the like can be added to the medium. When the transformant obtained by transformation with an expression vector having trp promoter inducible with indoleacetic acid (IAA), IAA or the like can be added to the medium.

The culture conditions for the transformant are not particularly limited as long as the conditions do not hinder the productivity of the modified Nampt of interest and the growth of the host. Usually, the culture is performed at 10°C to 40°C, preferably 20°C to 37°C, for 5 to 100 hours. pH is adjusted using an inorganic or organic acid, an alkali solution, or the like and adjusted to, for example, pH 6 to 9 for a *Rhodococcus* bacterium.

Examples of the culture method include solid culture, static culture, shake culture, and culture under aeration and stirring. Particularly, in the case of culturing the transformant of a *Rhodococcus* bacterium, the culture is preferably performed under aerobic conditions by shake culture or culture under aeration and stirring (jar fermenter).

As a result of culture under the culture conditions described above, the modified Nampt of the present invention can be accumulated at a high yield in the cultures, i.e., at least any of a culture supernatant, cultured cells, cultured microbial cells, or homogenates of the cells or the microbial cells.

When the modified Nampt is produced within the microbial cells or the cells thus cultured, the modified Nampt of interest can be collected by homogenizing the microbial cells or the cells. For example, high-pressure treatment using a French press or a homogenizer, sonication, grinding treatment using glass beads or the like, enzymatic treatment using lysozyme, cellulase or pectinase, etc., freezing-thawing treatment, hypotonic solution treatment, or bacteriolysis induction treatment using a phage can be used as a method for homogenizing the microbial cells or the cells.

If necessary, homogenized residues (including an insoluble fraction of cell extracts) can be removed from the microbial cells or the cells thus homogenized. Examples of the method for removing the residues include centrifugation and filtration. If necessary, residue removal efficiency may be enhanced using a flocculant, a filter aid, or the like. The supernatant obtained after the removal of the residues is a soluble fraction of cell extracts and can be used as a partially purified modified Nampt solution.

When the modified Nampt is produced within the microbial cells or the cells, the microbial cells or the cells themselves may be recovered by centrifugation, membrane separation, or the like and used in an unhomogenized state.

When the modified Nampt is produced outside the microbial cells or the cells, the culture solution is directly used or the microbial cells or the cells are removed therefrom by centrifugation, filtration, or the like. Then, the modified Nampt may be collected, if necessary, from the cultures by extraction or the like through ammonium sulfate precipitation, further dialyzed, if necessary, and isolated or purified by use of any various chromatography techniques (gel filtration, ionexchange chromatography, affinity chromatography, etc.).

The production yield of the Nampt obtained by the culture of the transformant can be confirmed by, but not particularly limited to, SDS-PAGE (polyacrylamide gel electrophoresis), Nampt activity measurement, or the like per unit, for example, per culture solution, per wet weight or dry weight of the microbial cells, or per protein in a crude enzyme solution. Those skilled in the art can perform SDS-PAGE by using a method known in the art. The value of the activity mentioned above can be applied to the Nampt activity.

In addition to the methods described above, the modified Nampt may be produced by adopting a cell-free protein synthesis system. The cell-free protein synthesis system is a system of synthesizing a protein in an artificial container such as a testing tube using cell extracts. The cell-free protein synthesis system used in the present invention also includes a cell-free transcription system of synthesizing RNA with DNA as a template.

In this case, an organism corresponding to the host described above corresponds to an organism from which the following cell extracts are derived. In this context, eukaryotic cell-derived or prokaryotic cell-derived extracts, for example, wheat germ or *Escherichia coli* extracts, can be used as the cell extracts. These cell extracts may or may not be concentrated.

The cell extracts can be obtained by, for example, ultrafiltration, dialysis, or polyethylene glycol (PEG) precipitation. In the present invention, the cell-free protein synthesis may be performed using a commercially available kit. Examples of such a kit include reagent kit PROTEIOS(TM) (Toyobo Co., Ltd.), TNT(TM) System (Promega Corp.), synthesis apparatus PG-Mate(TM) (Toyobo Co., Ltd.), and RTS (Roche Diagnostics K.K.).

The modified Nampt obtained by cell-free protein synthesis as described above can be purified by chromatography appropriately selected as mentioned above.

### 6. Method for producing nicotinamide mononucleotide

### 6.1 Production of NMN using modified Nampt

The modified Nampt of the present invention can be used as an enzyme catalyst in matter production. For example, a NMN compound can be produced by contacting phosphoribosyl pyrophosphate (PRPP) and nicotinamide (NAM) with each other in the presence of the modified Nampt, and collecting formed nicotinamide mononucleotide (NMN) .

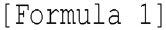

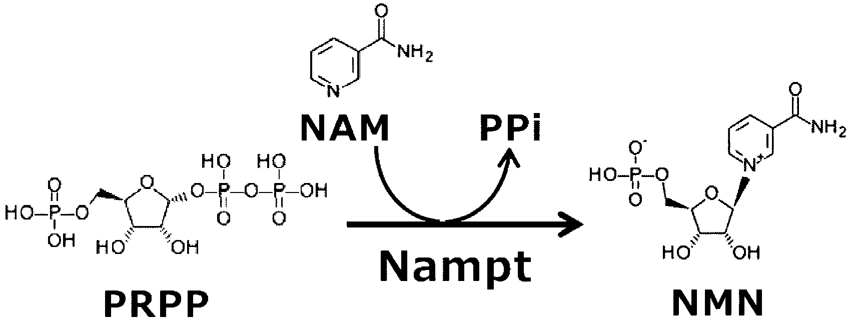

The separated or purified Nampt as well as cultures after culture of the transformant of the present invention, or a treated product of the cultures can be used as the enzyme catalyst. Examples of the treated product include cultured cells (transformants) contained in gels of acrylamide or the like, treated with glutaraldehyde, or supported by inorganic carriers such as resins, alumina, silica, zeolite and diatomaceous earth.

In this context, the "contact" means that the modified Nampt, PRPP and NAM are allowed to exist in the same reaction system or culture system, and includes, for example, the mixing of the separated or purified modified Nampt with PRPP and NAM, the addition of PRPP and NAM to a culture container of cells (transformants) expressing the modified Nampt gene, the culture of the cells in the presence of PRPP and NAM, and the mixing of extracts of the cells with PRPP and NAM.

The phosphoribosyl pyrophosphate can be produced, for example, from ribose-5-phosphate in the presence of phosphoribosyl pyrophosphate synthetase. Examples of the phosphoribosyl pyrophosphate synthetase include human (*Homo* sapiens)-derived phosphoribosyl pyrophosphate synthetase (NP_002755), *Bacillus subtilis-derived* phosphoribosyl pyrophosphate synthetase (BAA05286), *Bacillus caldolyticus-derived* phosphoribosyl pyrophosphate synthetase (CAA58682), *Arabidopsis thaliana*-derived phosphoribosyl pyrophosphate synthetase (Q680A5), and *Methanocaldococcus jannaschii-*derived phosphoribosyl pyrophosphate synthetase (Q58761). If necessary, mutant phosphoribosyl pyrophosphate synthetase may be used. Examples of the mutant phosphoribosyl pyrophosphate synthetase include Asp51His (substitution of ASP at position 51 by His; the same holds true for the description below), Asn113Ser, Leu128Ile, Aspl82His, Ala189Val, and Hisl92Gln mutants of human-derived Prs, and corresponding mutants of Prs derived from other organisms, for example, Asn120Ser (which corresponds to the Asn113Ser) and Leu135Ile (which corresponds to the Leu128Ile) mutants of *Bacillus subtilis*-derived Prs.

The ribose-5-phosphate can be produced from ribose in the presence of ribokinase. Natural ribokinase derived from any of various organisms, or mutant ribokinase prepared by modifying its amino acid sequence can be used as the ribokinase. Examples thereof include human (*Homo sapiens*)-derived ribokinase (NP_002755), yeast (*Saccharomyces cerevisiae*)*-*derived ribokinase (P25332), *Bacillus* subtilis-derived ribokinase (P36945), *Escherichia coli*-derived ribokinase (AAA51476), and *Haemophilus influenzae-*derived ribokinase (P44331).

### 6.2 Enhanced expression of enzyme

The method for producing NMN according to the present invention may be carried out in accordance with, for example, a method described in WO2020/129997. Specifically, the method comprises the step of contacting a transformant having enhanced expression of 3 enzymes Nampt, Prs and Ppk, a cell-free protein synthesis reaction solution caused to express the 3 enzymes, or a treated product thereof with R5P, NAM, ATP, and polyphosphate. Preferably, the method for producing NMN according to the present invention further comprises the step of contacting a transformant having enhanced expression of 2 enzymes Rbk and Ppk, a cell-free protein synthesis reaction solution caused to express the 2 enzymes, or a treated product thereof with a mixture containing ribose, ATP, and polyphosphate, as the step of producing the R5P. Specifically, in the present invention, NMN is produced by allowing predetermined enzymatic reaction to progress while exploiting ATP regeneration reaction.

Such a method for producing NMN is typically carried out by performing steps (1) to (3) given below (referred herein to as a first step, a second step and a third step, respectively) in order. These steps may be carried out by the same person or may be carried out by different persons. These steps may be continuously performed or may be performed in stages at intervals of a predetermined period between the steps.
(1) The step of preparing and culturing a transformant comprising a gene encoding each of enzymes Nampt, Prs, Rbk and Ppk, or performing protein synthesis reaction with a cell-free protein synthesis reaction solution containing a gene encoding each of the enzymes to express each of the enzymes (first step);
(2) the step of preparing a treated product, if necessary, from the transformant or the cell-free protein synthesis reaction solution that has undergone the first step (second step); and
(3) the step of contacting the transformant, the cell-free protein synthesis reaction solution, or the treated product thereof that has undergone the first or second step with each substrate compound (third step).

Hereinafter, the method for producing NMN according to the present invention will be described in more detail with reference to embodiments in which the first step, the second step and the third step are performed. However, the method for producing NMN according to the present invention may be performed by appropriately modified embodiments of the first step, the second step and the third step specifically described below, without departing from the spirit of the present invention.

### [First step]

The first step is the step of preparing and culturing a transformant comprising a gene encoding each of enzymes Nampt, Prs, Rbk and Ppk, or performing protein synthesis reaction with a cell-free protein synthesis reaction solution containing a gene encoding each of the enzymes to express each of the enzymes.

In the present embodiment, 4 enzymes, Nampt, Prs and Ppk and, optionally, Rbk, are used. All of Nampt, Prs, Ppk and Rbk are known enzymes. Those skilled in the art are readily capable of obtaining their amino acid sequences and the nucleotide sequences of genes encoding these enzymes. Each of the predetermined 4 enzymes may be a natural enzyme or may be a mutant enzyme preferably having an improved expression level or enzymatic activity, prepared by modifying the amino acid sequence of the natural enzyme as long as the enzyme can catalyze the reaction of interest. Any of various tags (proteins or peptides) may be added to each enzyme for the purpose of, for example, simplifying purification, promoting soluble expression, or detecting the enzyme with an antibody. Examples of the type of the tag include His tag (histidine tag), Strep(II)-tag, GST tag (glutathione-S-transferase tag), MBP tag (maltose binding protein tag), GFP tag (green fluorescence protein tag), SUMO tag (small ubiquitin-related (like) modifier tag), FLAG tag, HA tag, and myc tag. The 4 enzymes may be expressed as a fusion protein with each other.

Examples of the Prs include human (*Homo sapiens*)-derived Prs (NP_002755), *Bacillus subtilis*-derived Prs (BAA05286), *Bacillus caldolyticus-*derived Prs (CAA58682), *Arabidopsis thaliana*-derived Prs (Q680A5), and *Methanocaldococcus jannaschii-*derived Prs (Q58761). Particularly, when a substrate concentration within a production system is high over a long time, mutant Prs as described in WO2020/129997 may be used such that the formation of PRPP through the second reaction and the subsequent formation of NMN through the third reaction can be continued (i.e., the concentration of the final product NMN within the production system can be continuously elevated). Examples of the mutant Prs include Asp51His (substitution of ASP at position 51 by His; the same holds true for the description below), Asn113Ser, Leu128Ile, Aspl82His, Ala189Val, and Hisl92Gln mutants of human-derived Prs, and corresponding mutants of Prs derived from other organisms, for example, Asn120Ser (which corresponds to the Asn113Ser) and Leu135Ile (which corresponds to the Leu128Ile) mutants of *Bacillus subtilis*-derived Prs.

Natural Rbk derived from any of various organisms, or mutant Rbk prepared by modifying its amino acid sequence can be used as the Rbk. Examples thereof include human (*Homo sapiens*)-derived Rbk (NP_002755), yeast (*Saccharomyces cerevisiae*)*-*derived Rbk (P25332), *Bacillus subtilis*-derived Rbk (P36945), *Escherichia coli-*derived Rbk (AAA51476), and *Haemophilus influenzae-*derived Rbk (P44331).

In the present invention, the Ppk (EC number: 2.7.4.1) is an enzyme that is used for the reaction of regenerating ATP from ADP formed through the first reaction or AMP formed through the second reaction, and polyphosphate (ATP regeneration reaction).

The Ppk can be classified according to an amino acid sequence and difference in kinetics into two families, polyphosphate kinase 1 family (Ppk1) and polyphosphate kinase 2 family (Ppk2). Activity of regenerating ATP with polyphosphate as a substrate is higher in Ppk2 than in Ppk1. Thus, Ppk2 is preferably used as the Ppk according to the present invention.

The Ppk2 can be further classified into three subfamilies, class 1, class 2 and class 3. Ppk2 of class 1 and Ppk2 of class 2 catalyze the reaction of forming ATP by the phosphorylation of ADP, and the reaction of forming ADP by the phosphorylation of AMP, respectively. By contrast, Ppk2 of class 3 can catalyze both the AMP phosphorylation reaction and the ADP phosphorylation reaction and can therefore form ATP from AMP by itself.

A combination of Ppk2 class 1 for regenerating ATP from ADP, and Ppk2 class 2 for regenerating ADP from AMP can be used as the Ppk according to the present invention. Alternatively, only Ppk2 class 1 or Ppk1 for regenerating ATP from ADP may be used as the Ppk according to the present invention when used in combination with adenylate kinase (which catalyzes the reaction of AMP + ATP -> 2ADP) for regenerating ADP from AMP. However, Ppk2 class 3 is preferably used because of its good efficiency that enables the enzyme to catalyze by itself both the reaction of regenerating ATP from ADP and the reaction of regenerating ATP from AMP. In the case of using such Ppk, Ppk for the first reaction and Ppk for the second reaction can be used as common Ppk.

Examples of the Ppk2 class 3 include *Deinococcus radiodurans-derived* Ppk2 (NP_293858), *Paenarthrobacter* aurescens-derived Ppk2 (ABM08865), *Meiothermus rube-*derived Ppk2 (ADD29239), *Deinococcus geothermalis*-derived Ppk2 (WP_011531362), and *Thermosynechococcus elongatus-*derived Ppk2 (NP_682498). Examples of the Ppk2 class 1 include *Rhodobacter sphaeroides*-derived Ppk2 (CS253628), *Sinorhizobium meliloti-*derived Ppk2 (NP_384613), *Pseudomonas aeruginosa-*derived PA0141 (NP_248831), *Pseudomonas aeruginosa-*derived PA2428 (NP_251118), and *Francisella tularensis*-derived Ppk2 (AJI69883). Examples of the Ppk2 class 2 include *Pseudomonas aeruginosa-*derived PA3455 (NP_252145). Examples of the adenylate kinase include *Bacillus cereus*-derived adenylate kinase (AAP07232).

### [Second step]

The second step is the step of preparing a treated product, if necessary, from the transformant or the cell-free protein synthesis reaction solution that has undergone the first step. Examples of the treated product of the transformant include dormant microbial cells, microbial cells having improved membrane permeability, inactivated microbial cells, and homogenized microbial cells prepared from the transformant. The treated product of the present invention also includes cell-free extracts and purified enzymes prepared from the homogenized microbial cells. Examples of the treated product of the cell-free protein synthesis reaction solution include purified enzymes prepared from the cell-free protein synthesis reaction solution. The treated product of the present invention also includes stabilized products obtained by the stabilization treatment of the transformant, the cell-free protein synthesis reaction solution and their treated products.

The dormant microbial cells can be prepared by use of a method known in the art. The dormant microbial cells mean microbial cells in a state where the proliferation thereof has been substantially terminated. Specifically, the dormant microbial cells can be prepared by recovering the transformant allowed to proliferate by culture from the medium, and then suspending the transformant in a buffer solution or the like free of a carbon source, etc. easily utilizable by the transformant, by freezing the recovered transformant, or by drying and pulverizing the recovered transformant. The method for recovering the transformant from the medium may be any method. Examples thereof include a method using centrifugation, and a method using membrane filtration. The centrifugation is not particularly limited as long as centrifugal force can be supplied to precipitate the transformant. Cylindrical type, separator plate type, or the like can be used. The centrifugal force can be on the order of, for example, 500 G to 20,000 G. The membrane filtration may be performed using any of a microfiltration (MF) membrane and an ultrafiltration (UF) membrane as long as the transformant can be recovered from the medium. Any buffer solution may be used for suspending the transformant as long as the buffer solution substantially terminates the proliferation of the transformant and maintains the functions of each predetermined enzyme. For example, a phosphate buffer solution, an acrylate buffer solution, a Tris (tris(hydroxymethyl)aminomethane)-HCl buffer solution, HEPES (2-(4-(2-Hydroxyethyl)-1-piperazinyl)ethanesulfonic acid), or Good's buffers can be used. In the case of freezing the transformant, the transformant can be frozen in a state where the majority of water has been removed by an operation such as the centrifugation described above, or in a state where the transformant is suspended in an appropriate buffer solution. The freezing temperature differs depending on a component of the buffer solution to suspend the transformant and may be any temperature as long as the transformant can be substantially frozen at the temperature. The temperature can be in the range of, for example, -210°C to 0°C. In the case of drying the transformant, the drying method may be any method as long as the method substantially terminates the proliferation of the transformant and maintains the functions of each predetermined enzyme. Examples thereof include freeze drying and spray drying methods.

The microbial cells having improved membrane permeability can be prepared by use of a method known in the art. For example, the treatment of the transformant with an organic solvent or a surfactant facilitates a substrate or a product permeating the cell membrane or cell wall of the transformant. The type of the organic solvent or the surfactant used is not particularly limited as long as the organic solvent or the surfactant improves membrane permeability and maintains the functions of each predetermined enzyme. Examples of the organic solvent include toluene and methanol. Examples of the surfactant include Triton-X 100 and benzethonium chloride.

The inactivated microbial cells can be prepared by use of an approach known in the art. The preparation can be performed by, for example, chemical treatment or heating treatment. In the chemical treatment, for example, a cationic surfactant such as benzethonium chloride, cetyl pyridinium chloride, methyl stearoyl chloride, or cetyl trimethylammonium bromide, or a zwitterionic surfactant such as alkyldiaminoethylglycine hydrochloride can be used. Other examples of such a chemical include alcohols such as ethanol, thiols such as 2-mercaptoethanol, amines such as ethylenediamine, and amino acids such as cysteine, ornithine, and citrulline. For the heating treatment, heat treatment can be performed using a temperature and a time that do not inactivate the enzyme of interest.

The homogenized microbial cells can be prepared by use of an approach known in the art. Examples thereof include sonication, high-pressure treatment using a French press or a homogenizer, grinding treatment using a bead mill, collision treatment using an impact breaking apparatus, enzymatic treatment using lysozyme, cellulase, pectinase, or the like, freezing-thawing treatment, hypotonic solution treatment, and bacteriolysis induction treatment using a phage. Any of these methods can be used singly or in combinations, if necessary. In the case of homogenizing cells in an industrial scale, it is preferred to perform, for example, high-pressure treatment, grinding treatment, collision treatment, or any of these treatments combined with enzymatic treatment, in consideration of operability, a recovery rate, cost, etc.

In the case of performing grinding treatment using a bead mill, the beads used have, for example, a density of 2.5 to 6.0 g/cm³ and a size of 0.1 to 1.0 mm, and the mill can usually be packed with appropriately 80 to 85% of the beads for homogenization. Any of a batch system and a continuous system can be adopted as an operating system.

In the case of performing high-pressure treatment, the treatment pressure is not particularly limited as long as the recovery rate of the protein of interest from cells is sufficiently high. The homogenization can be performed at a pressure on the order of, for example, 40 to 200 MPa, preferably 60 to 150 MPa, more preferably 80 to 120 MPa. If necessary, the efficiency of homogenization and operation may be enhanced through multistage treatment by placing apparatuses in series or using an apparatus having a plurality of stage structures. It is usually preferred to perform cooling treatment, if necessary, because temperature elevation of 2 to 3°C occurs around a treatment pressure of 10 MPa.

In the collision treatment, cells can be efficiently homogenized, for example, by preparing cell slurry into frozen fine particles (e.g., 50 µm or smaller) in advance by rapid spray freezing treatment (freezing rate: e.g., several thousands of °C per minute), and allowing the particles to collide with a collision plate with a highspeed (e.g., approximately 300 m/s) carrier gas.

The cell-free extracts can be prepared by removing homogenized residues (insoluble fraction containing cell membranes, cell walls, and the like) from the homogenized microbial cells. The removal of the homogenized residues can be performed by an approach known in the art. For example, centrifugation, membrane filtration, or filtration through filter cloth can be used. The centrifugation operation can be performed as mentioned above. When the homogenized residues of the transformant are fine and does not readily precipitate, residue precipitation efficiency may be enhanced, if necessary, using a flocculant or the like. The membrane filtration can also be performed as mentioned above. When the homogenized residues of the transformant are fine, particularly, an ultrafiltration (UF) membrane can be used. In the case of performing filtration through filter cloth, the filtration can be performed in combination with a filter aid or a flocculant. Examples of the filter aid include diatomaceous earth, cellulose powders, and active carbon. Examples of the flocculant include cationic flocculants, anionic flocculants, amphoteric flocculants, and nonionic flocculants. By any of the operations described above, a supernatant containing no microbial cell can be recovered as a cell-free supernatant to prepare cell-free extracts containing each predetermined enzyme.

The purified enzyme can be prepared by any of general biochemical methods, for example, ammonium sulfate precipitation, various chromatography techniques (e.g., gel filtration chromatography (Sephadex column, etc.), ion-exchange chromatography (DEAE-Toyopearl, etc.), affinity chromatography (TALON Metal Affinity Resin, etc.), hydrophobic chromatography (butyl Toyopearl, etc.), and anionic chromatography (MonoQ column, etc.)), and SDS polyacrylamide gel electrophoresis each used singly or in appropriate combination.

In the present invention, the transformant, the cell-free protein synthesis reaction solution, or the treated product thereof mentioned above may be subjected to stabilization treatment (stabilized product) for use. The stabilization treatment may be any treatment as long as the treatment improves the stability of each predetermined enzyme against an environmental factor (temperature, pH, chemical concentration, etc.) or its stability during preservation as compared with an untreated state. Examples thereof include containment in gels of acrylamide or the like, treatment with aldehydes such as glutaraldehyde (including CLEA: cross-linked enzyme aggregate), and supporting treatment onto inorganic carriers (alumina, silica, zeolite, diatomaceous earth, etc.).

The substrate or the product used in the present invention has relatively high polarity. Thus, it is possible that cell membranes or cell walls limit the mass transfer thereof. Thus, in the present invention, particularly, homogenized microbial cells, cell-free extracts, a purified enzyme, or a stabilized product thereof is preferably used from the viewpoint of the permeability of the substrate or the product.

The transformant, the cell-free protein synthesis reaction solution, or the treated product thereof mentioned above may be preserved under any condition as long as its enzymatic activity is retained. If desired, the solution thereof may be frozen under suitable conditions (e.g., at -80°C to -20°C for 1 day to 1 year) and preserved until use (execution of the third step).

As mentioned above, in the case of combining transformants separately comprising respective genes encoding predetermined enzymes, for example, in the case of preparing and combining a transformant having enhanced expression of Nampt and Prs and a transformant having enhanced expression of Ppk, (i) these transformants may be separately subjected to each treatment and the treated products thereof can be mixed, or (ii) these transformants may be mixed and then subjected in one portion to each treatment.

### [Third step]

The third step is the step of contacting the transformant or the cell-free protein synthesis reaction solution that has undergone the first step or, optionally, the treated product thereof that has undergone the second step with each substrate serving as a starting material for enzymatic reaction. In this step, the second reaction mediated by Prs and the third reaction mediated by Nampt are performed by conjugation with ATP regeneration reaction mediated by Ppk. In the second reaction mediated by Prs, the substrate is phosphorylated with ATP as a phosphate source. In the third reaction mediated by Nampt, the Nampt is autophosphorylated through the ATP hydrolytic activity of the Nampt itself. Therefore, ATP is consumed. Thus, both the reactions can be performed by conjugation with the ATP regeneration reaction so that the reactions progress efficiently while compensating for the consumed ATP. Since phosphoribosyl pyrophosphate (PRPP) which is a product of the second reaction mediated by Prs is a relatively unstable compound, the third reaction mediated by Nampt can be performed immediately after PRPP formation by performing the second reaction and the third reaction in the same system. In the present invention, ATP is not depleted because ATP is regenerated from ADP and/or AMP through the ATP regeneration reaction. However, a moderate amount of ATP needs to be added into a reaction solvent according to an ATP concentration to be maintained during reaction. In this respect, ADP or AMP may be added into the reaction solvent, if necessary, instead of ATP. The added ADP or AMP is immediately used in the ATP regeneration system to regenerate ATP in the system. This is substantially the same state as that where a moderate amount of ATP has been added. Alternatively, a mixture containing these components at an arbitrary ratio may be added thereto.

The second reaction and the third reaction may be combined, if necessary, with the first reaction mediated by Rbk conjugated with the ATP regeneration reaction mediated by Ppk. In the case of combining the reactions, the first reaction mediated by Rbk may be first performed, followed by the second reaction mediated by Prs and the third reaction mediated by Nampt using the reaction solution as a starting material, or the first reaction mediated by Rbk, the second reaction mediated by Prs and the third reaction mediated by Nampt may be performed in the same reaction system.

The second reaction mediated by Prs and the third reaction mediated by Nampt are performed by contacting a transformant having enhanced expression of 3 enzymes Nampt, Prs and Ppk, a cell-free protein synthesis reaction solution caused to express the 3 enzymes, or a treated product thereof with R5P, NAM, ATP, and polyphosphate.

The first reaction mediated by Rbk is performed by contacting a transformant having enhanced expression of 2 enzymes Rbk and Ppk, a cell-free protein synthesis reaction solution caused to express the 2 enzymes, or a treated product thereof with ribose, ATP, and polyphosphate.

The starting materials for use in the third step may be purchased from general suppliers or may be synthesized through reaction by users themselves. For example, as for R5P, a commercially available product may be used, or R5P synthesized through the first reaction mediated by Rbk, i.e., by contacting a transformant having enhanced expression of 2 enzymes Rbk and Ppk, a cell-free protein synthesis reaction solution caused to express the 2 enzymes, or a treated product thereof with ribose and polyphosphate, is preferably used from the viewpoint of starting material cost.

Polyphosphate, one of the starting materials, is known to have various chain lengths. The chain length of the polyphosphate used in the present invention may be any chain length as long as the ATP regeneration reaction can be efficiently performed. The chain length is preferably on the order of 3 to 100, more preferably on the order of 3 to 30, from the viewpoint of the viscosity of a solution upon dissolution, or cost.

In the third step, if necessary, a compound other than the starting materials may be contacted with a transformant having enhanced expression of each predetermined enzyme, a cell-free protein synthesis reaction solution caused to express each predetermined enzyme, or a treated product thereof, or may be contained in a reaction solvent (within the production system). For example, a metal ion such as a magnesium ion is suitably contained therein as a component for allowing each enzyme to exert functions. Also, a buffer component is preferably contained therein.

When the transformant used is substantially alive, i.e., maintains the ability of cells to proliferate, the reaction is preferably performed under conditions that do not substantially allow the transformant to proliferate. The reaction under such conditions can be expected to produce the product of interest at a high yield without utilizing the substrate or the product of this reaction in the proliferation of the transformant, or without degrading the substrate or the product. The conditions that do not substantially allow the transformant to proliferate may be any condition as long as the condition does not substantially increase the number of transformants within the reaction system. Examples thereof include reaction that is performed in a solution free of a carbon source (glucose, etc.) easily utilizable by the transformant.

The amount of the transformant having enhanced expression of each predetermined enzyme, the cell-free protein synthesis reaction solution caused to express each predetermined enzyme, or the treated product thereof, more specifically, the respective amounts of Nampt, Prs, Rbk and Ppk, contained in the reaction solvent (within the production system) can be appropriately adjusted. Likewise, the amounts of R5P, NAM, ATP, polyphosphate, ribose, and other starting materials to be optionally used, contained in the reaction medium can be appropriately adjusted. The concentration of each of these substances in the reaction solvent (within the production system) is as follows.

The concentration of Nampt is, for example, 1 µg/L to 5 g/L. The concentration of Prs is, for example, 1 µg/L to 1 g/L. The concentration of Rbk is, for example, 1 µg/L to 1 g/L. The concentration of Ppk is, for example, 1 µg/L to 5 g/L. The concentration of each enzyme in the reaction solvent may be adjusted to the range described above by appropriately adjusting the amount of the transformant having enhanced expression of each predetermined enzyme, the cell-free protein synthesis reaction solution caused to express each predetermined enzyme, or the treated product thereof added to the reaction solvent.

The concentration of R5P is, for example, 1 µg/L to 100 g/L. The concentration of ribose is, for example, 1 µg/L to 100 g/L. The concentration of NAM is, for example, 1 µg/L to 500 g/L. The concentration of ATP is, for example, 1 µg/L to 100 g/L. The concentration of polyphosphate is, for example, 1 µg/L to 200 g/L. The concentration of each starting material in the reaction solvent may be adjusted to the range described above by adjusting, for example, the amounts of these starting materials added to the reaction solvent. The addition to the reaction solvent depends on a starting material, and a predetermined amount of the starting material may be added in one portion at the start of the third step, or predetermined amounts of the starting material may be sequentially added at the start of the third step and/or at suitable stages during the third step.

Conditions other than the concentration of each substance as mentioned above, for example, a temperature and a time, for allowing enzymatic reaction to progress can also be appropriately adjusted. The reaction temperature is preferably adjusted within a range that optimizes the catalysis efficiency of each enzyme. The reaction time can be a time required for the amount of NMN, the compound of interest, formed to reach a predetermined amount.

The formed NMN can be recovered from the production system in accordance with a routine method and appropriately concentrated and purified. Any method may be used as a recovery or purification method as long as the method can improve the purity of NMN and can efficiently recover NMN. For example, methods described below can be used. In the case of performing reaction using microbial cells after NMN synthesis reaction, the microbial cells can be removed by an approach such as centrifugation or membrane filtration. Alternatively, in the case of performing reaction using cell-free extracts or a purified enzyme, proteins, etc. can be removed, for example, by filtration through an ultrafiltration membrane or precipitation by the addition of perchloric acid or the like, followed by centrifugation. In the case of performing treatment with perchloric acid, pH is brought back to weakly acidic pH with potassium hydroxide or the like, and the resulting precipitates of potassium perchlorate are removed by centrifugation again. After removal of the microbial cells or the proteins, washing treatment can be performed by active carbon adsorption, if necessary. The aqueous solution containing NMN is contacted with active carbon so that the NMN is adsorbed thereon. The active carbon with the NMN adsorbed thereon can be filtered through a filter paper or the like and then washed with a solvent such as isoamyl alcohol to remove given impurities. Subsequently, the resulting product can be further purified by treatment with an anion-exchange resin. The solution containing NMN can be passed through an anion-exchange resin such as Dowex, followed by the elution of the adsorbed NMN with water. The pH of the obtained aqueous NMN solution is further rendered acidic, and NMN can be obtained as precipitates by the addition of acetone in a large amount. The precipitates can be dried to obtain purified NMN.

The present invention can be practiced such that the total number of moles of ATP, ADP and AMP to be added to the reaction system is equal to or less than 0.5 equivalents of the number of moles of NMN to be formed. Any method may be used as an approach for performing this as long as the method can consequently reduce the amount of ATP, etc. used to be added for NMN production. For example, the following approaches can be carried out each singly or in appropriate combination.
(1) Conjugation with an ATP regeneration system
(2) Coexistence of PPase
(3) Utilization of bacterium-derived Nampt
(4) Utilization of a host from which unnecessary genes have been disrupted or deleted
(5) Suitable substrate concentration

### 6.3 Enhanced expression of Nampt in presence of PPase

The method for producing NMN according to the present invention may comprise the step of contacting a transformant having enhanced expression of Nampt, a cell-free protein synthesis reaction solution caused to express the enzyme, or a treated product thereof with NAM and PRPP in the presence of PPase.

The step of the present embodiment is carried out by performing the first step, the second step and the third step in order in the same manner as in the embodiment of 6.2 except that: the first step and the second step are steps for preparing at least a transformant having enhanced expression of Nampt, a cell-free protein synthesis reaction solution caused to express the enzyme, or a treated product thereof and also preparing a transformant having enhanced expression of PPase or a microbe or the like expressing PPase as an endogenous enzyme whose expression is not particularly enhanced, a cell-free protein synthesis reaction solution caused to express the enzyme, or a treated product thereof; and in the third step, the transformants, the cell-free protein synthesis reaction solutions or the treated products thereof that have undergone such a first step and a second step can be contacted with at least NAM and PRPP.

The PPase (EC number: 3.6.1.1) is an enzyme that hydrolyzes pyrophosphate into two molecules of phosphate. In the second embodiment of the method for producing NMN according to the present invention, the third reaction is performed in the presence of the PPase so that the third reaction can progress very efficiently.

In the third reaction mediated by Nampt, pyrophosphate is formed as a by-product together with NMN. From a general standpoint of enzymatic reaction, it is predicted that the by-product pyrophosphate is degraded into phosphate by the addition of PPase to the third reaction system to accelerate the reaction toward NMN formation. However, this is not always obvious for Nampt. This is because the Nampt reaction may not continuously progress if pyrophosphate is degraded. It is known that Nampt is activated by ATP hydrolysis and autophosphorylation. For continuing the third reaction mediated by Nampt, it is necessary to dephosphorylate the autophosphorylated Nampt on a reaction basis. The pyrophosphate is reportedly a trigger substance for the dephosphorylation (Biochemistry 47, 11086-11096 (2008)). Thus, if the pyrophosphate is removed with PPase, it is perfectly possible that the Nampt dephosphorylation does not occur and the reaction is not continued. However, in the second embodiment of the method of the present invention, the third reaction can be markedly accelerated by performing the third reaction in the presence of PPase.

Examples of the PPase include yeast-derived PPase (P00817), *Escherichia coli*-derived PPase (NP_418647), *Bacillus subtilis*-derived PPase (P37487), *Thermus* thermophilus-derived PPase (P38576), *Streptococcus gordonii-*derived PPase (P95765), and *Streptococcus mutans*-derived PPase (068579).

The PPase may be in any form and may be prepared by any method as long as the PPase is capable of being added to the third reaction system. Specifically, the PPase is first expressed, in the same manner as in the first step in the first embodiment of the method of the present invention, by preparing and culturing a transformant comprising a gene encoding the enzyme, or performing protein synthesis reaction with a cell-free protein synthesis reaction solution containing a gene encoding the enzyme. Since the PPase is expressed in a given amount as an enzyme necessary for survival in usual microbes or the like, such a microbe or the like in which the expression of the enzyme is not particularly enhanced can be cultured and directly used. Subsequently, a treated product can be prepared from the transformant, the microbe or the like in which the expression is not enhanced, or the cell-free protein synthesis reaction solution that has undergone the first step, in the same manner as in the second step in the first embodiment of the method of the present invention. Alternatively, a commercially available purified enzyme of PPase may be used as one form of the treated product. Examples of the commercially available purified enzyme of PPase include a purified enzyme of yeast-derived PPase from Sigma-Aldrich Co., LLC (product No. 10108987001).

The method for producing NMN according to the second embodiment of the present invention can be carried out by contacting a transformant having enhanced expression of Nampt, a cell-free protein synthesis reaction solution caused to express the enzyme, or a treated product thereof with NAM and PRPP in the presence of the PPase obtained as described above. The second embodiment of the method of the present invention enables the third reaction to progress very efficiently and can efficiently produce NMN. The third reaction according to the second embodiment of the method of the present invention may be performed singly or may be performed in combination with one or more of the first reaction, the second reaction and the ATP regeneration reaction in the same reaction system. In other words, the first embodiment of the method of the present invention and the second embodiment of the method of the present invention may be carried out as an integrated method for producing NMN by performing the third reaction in the first embodiment of the method of the present invention as the third reaction defined in the second embodiment of the method of the present invention.

When the transformant used is substantially alive, i.e., maintains the ability of cells to proliferate, the reaction is preferably performed under conditions that do not substantially allow the transformant to proliferate. The reaction under such conditions can be expected to produce the product of interest at a high yield without utilizing the substrate or the product of this reaction in the proliferation of the transformant, or without degrading the substrate or the product. The conditions that do not substantially allow the transformant to proliferate may be any condition as long as the condition does not substantially increase the number of transformants within the reaction system. Examples thereof include reaction that is performed in a solution free of a carbon source (glucose, etc.) easily utilizable by the transformant.

A particularly preferred form of the second step according to the present invention employs a purified enzyme as the treated product. Use of the purified enzyme can suppress the degradation or side reaction of a reactant (substrate) or a product. Therefore, the accelerating effect on the third reaction according to the present invention can be more enjoyed.

### 6.4 Disruption of particular enzyme

The method for producing NMN according to the present invention may comprise the step of contacting a transformant having enhanced expression of nicotinamide phosphoribosyltransferase (Nampt) in which a gene encoding an enzyme classified into EC No. shown in (d) EC 3.5.1.42 and a gene encoding at least one enzyme classified into EC No. shown in any of the following (a), (c), (g), (h) and (i) are disrupted or deleted, or a treated product thereof with at least nicotinamide (NAM).
(a) EC 3.1.3.5
(c) EC 2.4.2.1
(g) EC 3.2.2.1
(h) EC 3.2.2.3
(i) EC 3.2.2.14

The step of the present embodiment is carried out by performing the first step, the second step and the third step in order in the same manner as in the embodiment of 6.2 except that: the first step and the second step are steps for preparing a transformant having enhanced expression of nicotinamide phosphoribosyltransferase (Nampt) in which a gene encoding an enzyme classified into each EC No. is disrupted or deleted, or a treated product thereof; and in the third step, the transformant or the treated product thereof that has undergone such a first step and a second step can be contacted with at least NAM.

### 6.5 Reduction in amount of ATP used

The method for producing NMN according to the present invention may be performed such that the total number of moles of ATP, ADP and AMP to be added to the reaction system for NMN production is equal to or less than 0.5 equivalents of the number of moles of NMN to be formed.

ATP is not essential for the Nampt-catalyzed reaction itself. However, as mentioned above, Nampt has ATP hydrolytic activity, and through the hydrolysis of ATP, Nampt is autophosphorylated so that enzymatic parameters or chemical equilibrium is shifted in favor of NMN formation. Thus, in the presence of ATP in a sufficient amount within the third reaction system, 1 mol or more of ATP is substantially used per mol of PRPP to be formed in association with the formation of NMN from PRPP. Specifically, 2 mol or more in total of ATP is used per mol of NMN to be formed in the production of NMN through the second reaction and the third reaction with R5P as a starting material, and 3 mol or more in total of ATP is used per mol of NMN to be formed in the production of NMN through the first reaction, the second reaction and the third reaction with ribose as a starting material.

Since ATP is an expensive compound, it is desirable to minimize the amount of ATP used for inexpensive production of NMN. Specifically, in the method for producing NMN according to the present invention, the total number of moles of ATP, ADP and AMP to be added to the reaction system for NMN production is preferably equal to or less than 0.5 equivalents of the number of moles of NMN to be formed.

Specifically, the total number of moles of ATP, ADP and AMP is set to be equal to or less than 0.5 equivalents of the number of moles of NMN to be formed by performing the following approaches each singly or in appropriate combination.

### (1) Conjugation with an ATP regeneration system

The total number of moles of ATP, ADP and AMP to be added to the reaction system for NMN production can be reduced by conjugating a system that can regenerate ATP from ADP or AMP formed as a by-product with a NMN formation reaction system involving at least the second reaction and the third reaction. The ATP regeneration system may be any system as long as the system can regenerate ATP from AMP and ADP. Examples thereof include a system using Ppk with polyphosphate as a phosphate source, a system using pyruvate kinase with phosphoenolpyruvate as a phosphate source, and a system using creatine phosphate kinase with creatine phosphate as a phosphate source. A system using Ppk with polyphosphate as a phosphate source is preferred from the viewpoint of phosphate source cost. NMN formation reaction conjugated with the ATP regeneration system using polyphosphate and Ppk can specifically be carried out as described in the third step in the first embodiment of the method of the present invention.

### (2) Coexistence of PPase

The by-product pyrophosphate is degraded into phosphate with PPase coexisting in the NMN formation reaction system to accelerate the reaction toward NMN formation. As a result, the total number of moles of ATP, ADP and AMP to be added to the reaction system for NMN production can be reduced. NMN formation reaction with coexisting PPase can specifically be carried out as described in the second embodiment of the method of the present invention.

### (3) Utilization of bacterium-derived Nampt

Bacterium-derived Nampt is used as the enzyme Nampt which catalyzes the third reaction so that the formation of NMN progresses efficiently. As a result, the total number of moles of ATP, ADP and AMP to be added to the reaction system for NMN production can be reduced. The bacterium-derived Nampt described in the first step in the first embodiment of the method of the present invention can be utilized.

### (4) Utilization of host from which unnecessary genes have been disrupted or deleted

In the case of performing NMN formation reaction using a transformant, dormant microbial cells, microbial cells having improved membrane permeability, inactivated microbial cells, or homogenized microbial cells prepared from the transformant, cell-free extracts prepared from the homogenized microbial cells, or a stabilized product obtained by the stabilization treatment thereof, a host obtained by the disruption or deletion of a gene causative of degradation or side reaction of a reactant (substrate) or a product can be used. Specifically, a host from which any one or more of the genes described in the first step in the first embodiment of the method of the present invention have been disrupted or deleted can be used. Preferably, any of the gene-disrupted or - deleted hosts described in the section 6.4 can be used.

### (5) Suitable substrate concentration

Improvement in NMN formation rate or the amount of NMN formed can be expected by elevating the concentration of ribose or NAM within the reaction system from the viewpoint of chemical equilibrium or the substrate affinity of an enzyme. As a result, the total number of moles of ATP, ADP and AMP to be added to the reaction system for NMN production can be reduced. On the other hand, a similar effect can be expected by elevating the concentration of ATP within the reaction system. However, the addition of ATP in an amount more than necessary increases the number of moles of ATP to be used for forming 1 mol of NMN without increase in the amount of NMN formed commensurate therewith. Specifically, NMN can be efficiently produced by adding a suitable amount of ATP to the reaction system. The number of moles of ATP to be added to the reaction system is preferably equal to or less than 1 equivalent, more preferably equal to or less than 0.5 equivalents, further preferably equal to or less than 0.1 equivalents, of the number of moles of NMN to be formed.

### [Examples]

Hereinafter, the present invention will be specifically described with reference to Examples. However, the present invention is not limited by these Examples.

### [Example 1] Preparation of Nampt library and screening

In this Example, the following enzymes described in WO2019/065876 were used as predetermined enzymes.
Nampt: derived from *Haemophilus ducreyi* (AAR87771)
Prs: Leu135Ile mutant derived from *Bacillus subtilis* (BsPrsL135I)
Ppk: derived from *Deinococcus radiodurans* (NP_293858)
Rbk: derived from *Saccharomyces cerevisiae* (P25332)
PPase: derived from *Escherichia coli* (NP_418647)

### (1) Construction of Nampt mutant library

Random mutations were introduced to nucleotide sequences corresponding to designated amino acid residues by using pEHdNampt (WO2019/065876, SEQ ID NO: 3) described in Table 1 as a template. Table 2 shows the names of primers for mutagenesis and SEQ ID NOs that represent their nucleotide sequences, and the names of Nampt mutants.

**[Table 2]**

| Mutagenesis primer | | | Nampt library name |
|---|---|---|---|
| Forward | Nampt_S247X-F | SEQ ID NO: 25 | HdNamptS247X |
| Reverse | Nampt_S247X-R | SEQ ID NO: 26 | |
| Forward | Nampt_E248X-F | SEQ ID NO: 27 | HdNamptE248X |
| Reverse | Nampt_E248X-R | SEQ ID NO: 28 | |
| Forward | Nampt_S253X-F | SEQ ID NO: 29 | HdNamptS253X |
| Reverse | Nampt_S253X-R | SEQ ID NO: 30 | |
| Forward | Nampt_V278X-F | SEQ ID NO: 31 | HdNamptS278X |
| Reverse | Nampt_V278X-R | SEQ ID NO: 32 | |
| Forward | Nampt_T281X-F | SEQ ID NO: 33 | HdNamptS281X |
| Reverse | Nampt_T281X-R | SEQ ID NO: 34 | |

Mutagenesis PCR reaction was performed using the reaction solution composition shown in Table 3 and the reaction conditions shown in Table 4.

**[Table 3]**

| Component | Final concentration |
|---|---|
| Template plasmid | 20pg/µL |
| Forward primer | 0.2µM |
| Reverse primer | 0.2µM |
| PrimeSTAR Max Premix (2x) (Takara Bio Inc.) | 1x |

**[Table 4]**

| Temperature | Time | The number of cycles |
|---|---|---|
| 98 °C | 10 sec | 30 |
| 55 °C | 5 sec | |
| 72 °C | 30 sec | |

After the completion of PCR reaction, the PCR product was purified using QIAquick PCR Purification Kit (Qiagen N.V.) according to the attached protocol. The purified PCR product was digested with DpnI (New England Biolabs Inc.), and *Escherichia coli* JM109 (Takara Bio Inc.) was transformed with the resulting reaction solution. Colonies that appeared were recovered, followed by plasmid extraction. Nucleotide sequences were confirmed using primers T7-PP (SEQ ID NO: 35) and T7-TP (SEQ ID NO: 36). Each plasmid correctly harboring the library was used as each Nampt library plasmid described in Table 2.

### (2) Expression of Nampt library and preparation of cell-free extract

Competent cells of an *Escherichia coli* (*E. coli*) BN8 strain (WO2019/065876) were thawed on ice, mixed with each plasmid DNA solution prepared in the section (1), and left standing on ice for 10 minutes. Heat shock was applied thereto at 42°C for 20 seconds. Then, the cells were ice-cooled again, and SOC medium was added thereto. The cells were shake-cultured at 37°C for 1 hour, then spread over LB agar medium (containing 50 mg/L kanamycin sulfate), and statically cultured overnight at 37°C. The obtained colonies were used as recombinants having enhanced expression of each enzyme. A single colony was placed in each well of a 96-well deep well plate containing 200 µL/well of LB medium (containing 50 mg/L kanamycin sulfate), and shake-cultured overnight at 37°C. 70 µL of the precultures was used for 630 µL of main culture (LB medium, containing 50 mg/L kanamycin sulfate and 0.5 mM (final concentration) IPTG) inoculation, followed by culture at 1250 rpm at 16°C for 24 hours or at 1250 rpm at 25°C for 18 hours. The cultured *Escherichia coli* was harvested by centrifugation (5000 rpm, 4°C, 15 min) to remove a supernatant. The recovered pellets were suspended in 200 µL of a lysis buffer (100 mM Kpi pH 8.5, 1 mg/ml lysozyme, 0.5 mg/ml polymyxin B, 0.05 mg/ml DNase) and shaken at 900 rpm at room temperature for 20 minutes. Insoluble matter was removed by centrifugation (4900 rpm, 10 min, 4°C), and the supernatant was used as cell-free extracts in reaction.

### (3) Expression of Prs, Rbk, and Ppk and preparation of cell-free extract

In the same manner as in the section (2), each plasmid pEBsPrs, pEScRbk, or pESDrPpk (WO2019/065876) was added to competent cells of an *Escherichia coli* (*E. coli*) BN8 strain, which were then left standing on ice for 10 minutes. Heat shock was applied thereto at 42°C for 20 seconds. Then, the cells were ice-cooled again, and SOC medium was added thereto. The cells were shake-cultured at 37°C for 1 hour, then spread over LB agar medium (containing 50 mg/L kanamycin sulfate), and statically cultured overnight at 37°C. The obtained colonies were used as recombinants having enhanced expression of each enzyme. A single colony was placed in a culture tube containing 2 mL of LB medium (containing 50 mg/L kanamycin sulfate), and shake-cultured overnight at 37°C. 1 mL of the precultures was used for 100 mL of main culture (LB medium, containing 50 mg/L kanamycin sulfate) inoculation, followed by shake culture at 37°C. When OD600 reached 0.8, IPTG was added thereto such that its final concentration was 0.5 mM, followed by culture at 1250 rpm at 25°C for 18 hours. The cultured *Escherichia coli* was harvested by centrifugation (8000 rpm, 4°C, 15 min) to remove a supernatant. The recovered pellets were suspended in 10 mL of a lysis buffer (100 mM Kpi pH 8.5, 1 mg/ml lysozyme, 0.5 mg/ml polymyxin B, 0.05 mg/ml DNase) and shaken at 900 rpm at room temperature for 20 minutes. Insoluble matter was removed by centrifugation (8000 rpm, 10 min, 4°C), and the supernatant was used as cell-free extracts in reaction.

### (4) NMN synthesis reaction

NMN synthesis reaction was performed using the cell-free extracts prepared in the sections (2) and (3). The amount of a reaction solution was set to 100 µL. Each reaction solution was prepared using the composition given below, and static reaction was performed at 37°C.

**[Table 5]**

| **Reagent** | **Final concentration** |
|---|---|
| Tricine (pH9.5) [mM] | 400 |
| KPB (pH8.5) [mM] | 50 |
| Ribose [mM] | 10 |
| Nicotinamide [mM] | 10 |
| Adenosine triphosphate (pH8.5) [mM] | 5 |
| MgCl₂ | 50 |
| Polyphosphate [mM] | 5 |

| **Cell-free extract** | **Final concentration** |
|---|---|
| ScRbk [g/L] | 0.15 |
| DrPpK [g/L] | 2.0 |
| BsPrs L135 [g/L] | 0.15 |
| HdNampt [g/L] | 1.0 |
| Ec PPase [g/L] | 0.1 |

At reaction times of 2 hours and 17 hours, 5 µL of each sample was transferred to a fresh plate. The resultant was diluted with 245 µL of a 50% aqueous methanol solution (1 mM EDTA) and subjected to HPLC analysis.

### (5) Analysis of NMN

The NMN synthesis reaction samples were analyzed by HPLC under the following conditions.
Column: Shodex Asahipak NH2P-50 4B
Guard column: Shodex Asahipak NH2P-50G 4A
Mobile phase: 25 mM aqueous ammonium formate solution
Flow rate: 1 ml/min (3.2-3.3 Mpa)
Detection: UV 261 nm
Column temperature: 40°C

The NMN conversion rate (%) of each sample was calculated from the ratio between NAM and NMN. As for a sample having a NMN conversion rate higher than that of the wild type, the mutation sites were sequenced.

### (6) Experimental results

Table 6 shows the relative activity of each mutant when the NMN conversion rate of the wild type was defined as 100%.

**[Table 6]**

| Mutant | Relative activity (%) |
|---|---|
| S247V | 121 |
| S247T | 144 |
| E248A | 110 |
| E248G | 120 |
| S253V | 115 |
| S253G | 135 |
| S253A | 160 |
| V278L | 118 |
| V278A | 132 |
| T281G | 134 |
| T281S | 173 |
| T281N | 178 |
| T281A | 156 |
| T281I | 170 |

From the results,improved versions of Nampt with higher activity than that of the wild-type Nampt were obtained from each library. Problems of conventional NMN synthesis using Nampt are that the Nampt is a rate-limiting enzyme and its starting material cost is high. Since the modified Nampt of the present invention has higher activity than that of the wild type as described above, the amount of the modified Nampt used can be reduced. This enables Nampt starting material cost to be reduced by approximately 30 to 40%.

### [Example 2] <Combination of good mutations in Nampt>

Good mutations of Example 1 were combined.

### (1) Construction of Nampt good mutation combination library

Random mutations were introduced to nucleotide sequences corresponding to designated amino acid residues by using degenerate codons and using pEHdNampt described in Table 1 as a template. Table 7 shows the names of primers for mutagenesis and SEQ ID NOs that represent their nucleotide sequences, and the names of Nampt mutants.

**[Table 7]**

| Mutagenesis primer | | | Nampt library name |
|---|---|---|---|
| Forward | Nampt_S247Z-F | SEQ ID NO: 37 | HdNampt |
| Reverse | Nampt_S247Z-R | SEQ ID NO: 38 | |
| Forward | Nampt_S253U-F | SEQ ID NO: 39 | |
| Reverse | Nampt_S253U-R | SEQ ID NO: 40 | S247Z_S253U_V278J_T281B |
| Forward | Nampt_V278J_T281B-F | SEQ ID NO: 41 | |
| Reverse | Nampt_V278J_T281B-R | SEQ ID NO: 42 | |

Mutagenesis PCR reaction, expression of Nampt libraries, preparation of cell-free extracts, and NMN synthesis reaction were performed by the same methods as in Example 1.

### (2) Experimental results

Table 8 shows the relative activity of each mutant when the NMN conversion rate of the wild type was defined as 100%.

**[Table 8]**

| Mutant | Relative activity (%) |
|---|---|
| S247T_V278A | 112 |
| S247T_T281S | 116 |
| S247T_S253G | 144 |
| S253G_T281S | 143 |
| S247T_S253T | 126 |
| S253A_T281S | 163 |
| V278G_T281S | 185 |

From the results, modified Nampt with higher activity than that of the wild-type Nampt was obtained by combining the good mutations. This enables Nampt starting material cost to be reduced by approximately 30 to 40%, as in the description above.

### [Example 3] <Introduction of good mutation to Nampt of different origin>

In this Example, the following enzymes were used as predetermined enzymes.
Nampt: derived from *Meiothermus ruber* (A0A0S7ALY5)
Nampt: derived from *Sphingopyxis* sp. C-1 (A0A0M9TZ33)

### (1) Construction of Nampt mutant

An expression plasmid for each enzyme was prepared as described below. DNA (consisting of the nucleotide sequence represented by each SEQ ID NO described in "Nucleotide sequence" of Table 1) encoding each enzyme protein consisting of the amino acid sequence represented by each SEQ ID NO described in "Amino acid sequence" of Table 1 was synthesized for each enzyme derived from an organism species described in "Origin" of Table 1, and cloned into the NdeI-XhoI site of an expression vector pET-26b(+) (Novagen) (gene synthesis was carried out by GenScript Biotech Corp.; codons were optimized for expression in *Escherichia coli*)*.* Each plasmid thus obtained was designated as an "expression plasmid" for each enzyme. Mutations were introduced to designated amino acid residues by using the plasmids as templates. Table 8 shows the names of primers for mutagenesis and SEQ ID NOs that represent their nucleotide sequences, and the names of Nampt mutants.

**[Table 9]**

| Mutagenesis primer | | | Mutant name |
|---|---|---|---|
| Forward | MrNampt_M230T-F | SEQ ID NO: 43 | MrNamptM230T |
| Reverse | MrNampt_M230T-R | SEQ ID NO: 44 | |
| Forward | MrNampt_E231G-F | SEQ ID NO: 45 | MrNamptE231G |
| Reverse | MrNampt_E231G-R | SEQ ID NO: 46 | |
| Forward | MrNampt_V264A-F | SEQ ID NO: 47 | MrNamptV264A |
| Reverse | MrNampt_V264A-R | SEQ ID NO: 48 | |
| Forward | SpNampt_E227G-F | SEQ ID NO: 49 | SpNamptE227G |
| Reverse | SpNampt_E227G-R | SEQ ID NO: 50 | |

Mutagenesis PCR reaction, expression of Nampt libraries, preparation of cell-free extracts, and NMN synthesis reaction were performed by the same methods as in Example 1. Table 9 shows the relative activity of each mutant when the NMN conversion rate of the wild type Nampt was defined as 100%.

**[Table 10]**

| Mutant | Relative activity (%) |
|---|---|
| MrNamptM230T | 255 |
| MrNamptE231G | 256 |
| MrNamptV264A | 692 |
| SpNamptE227G | 120 |

### [Industrial Applicability]

The present invention is useful in the industrial production of nicotinamide mononucleotide.

All publications, patents and patent applications cited herein are incorporated herein by reference in their entirety.

### [Free Text of Sequence Listing]

SEQ ID NO: 1: Motif sequence
SEQ ID NO: 2: Motif sequence
SEQ ID NO: 25: Primer (Nampt_S247X-F)
SEQ ID NO: 26: Primer (Nampt_S247X-R)
SEQ ID NO: 27: Primer (Nampt_E248X-F)
SEQ ID NO: 28: Primer (Nampt_E248X-R)
SEQ ID NO: 29: Primer (Nampt_S253X-F)
SEQ ID NO: 30: Primer (Nampt_S253X-R)
SEQ ID NO: 31: Primer (Nampt_V278X-F)
SEQ ID NO: 32: Primer (Nampt_V278X-R)
SEQ ID NO: 33: Primer (Nampt_T281X-F)
SEQ ID NO: 34: Primer (Nampt_T281X-R)
SEQ ID NO: 35: Primer (T7-PP)
SEQ ID NO: 36: Primer (T7-TP)
SEQ ID NO: 37: Primer (Nampt_S247Z-F)
SEQ ID NO: 38: Primer (Nampt_S247Z-R)
SEQ ID NO: 39: Primer (Nampt_S253U-F)
SEQ ID NO: 40: Primer (Nampt_S253U-R)
SEQ ID NO: 41: Primer (Nampt_V278J_T281B-F)
SEQ ID NO: 42: Primer (Nampt_V278J_T281B-R)
SEQ ID NO: 43: Primer (MrNampt_M230T-F)
SEQ ID NO: 44: Primer (MrNampt_M230T-R)
SEQ ID NO: 45: Primer (MrNampt_E231G-F)
SEQ ID NO: 46: Primer (MrNampt_E231G-R)
SEQ ID NO: 47: Primer (MrNampt_V264A-F)
SEQ ID NO: 48: Primer (MrNampt_V264A-R)
SEQ ID NO: 49: Primer (SpNampt_E227G-F)
SEQ ID NO: 50: Primer (SpNampt_E227G-R)

## Claims

1. A modified nicotinamide phosphoribosyltransferase (Nampt), comprising the amino acid sequence represented by the following SEQ ID NO: 1 and/or the amino acid sequence represented by the following SEQ ID NO: 2,
the modified Nampt having improved activity as compared with wild-type Nampt:
(a) SEQ ID NO: 1: S-[V/I]-P-A-X₁-X₂-H-S-[T/V/I]-[M/V/I]-X₃, and
(b) SEQ ID NO: 2: X₄-[S/I]-D-X₅
wherein at least one of X₁ to X₅ is different from the corresponding amino acid of the wild type; X₂ represents an amino acid other than A and Q; X₃ represents an amino acid other than M; and the parentheses [ ] represent any one of the amino acids within the parentheses [ ].

2. A modified Nampt, comprising the amino acid sequence represented by the following SEQ ID NO: 1 and/or the amino acid sequence represented by the following SEQ ID NO: 2,
the modified Nampt having improved activity as compared with wild-type Nampt:
(a) SEQ ID NO: 1: S-[V/I]-P-A-X₁-X₂-H-S-[T/V/I]-[M/V/I]-X₃, and
(b) SEQ ID NO: 2: X₄-[S/I]-D-X₅
wherein at least one of X₁ to X₅ is different from the corresponding amino acid of the wild type; X₁ represents any amino acid selected from neutral amino acids and aliphatic amino acids; X₂ represents any amino acid selected from aliphatic amino acids, acidic amino acids and neutral hydrophilic amino acids; X₃ represents any amino acid selected from nonpolar amino acids; X₄ represents any amino acid selected from aliphatic amino acids; X₅ represents any amino acid selected from amino acids other than aromatic amino acids; and the parentheses [ ] represent any one of the amino acids within the parentheses [ ].

3. A modified nicotinamide phosphoribosyltransferase (Nampt), comprising the amino acid sequence represented by the following SEQ ID NO: 1 and/or the amino acid sequence represented by the following SEQ ID NO: 2,
the modified Nampt having improved activity as compared with wild-type Nampt:
(a) SEQ ID NO: 1: S-[V/I]-P-A-X₁-X₂-H-S-[T/V/I]-[M/V/I]-X₃, and
(b) SEQ ID NO: 2: X₄-[S/I]-D-X₅
wherein at least one of X₁, X₄, and X₅ is different from the corresponding amino acid of the wild type; X₁ represents any amino acid selected from neutral amino acids and aliphatic amino acids; X₄ represents any amino acid selected from aliphatic amino acids; X₅ represents any amino acid selected from amino acids other than aromatic amino acids; X₂ and X₃ each represent any amino acid; and the parentheses [ ] represent any one of the amino acids within the parentheses [ ].

4. The modified Nampt according to any one of claims 1 to 3, wherein
when the amino acid sequence of the modified Nampt is aligned with the amino acid sequence represented by SEQ ID NO: 3,
the amino acid sequence represented by SEQ ID NO: 1 is located at positions corresponding to positions 243 to 253 of SEQ ID NO: 3, and
the amino acid sequence represented by SEQ ID NO: 2 is located at positions corresponding to positions 278 to 281 of SEQ ID NO: 3.

5. The modified Nampt according to any one of claims 1 to 4, wherein the modified Nampt has one or two or more substitutions selected from the following 1) to 5):
1) a substitution of the wild-type amino acid at X₁ by T,
2) a substitution of the wild-type amino acid at X₂ by G,
3) a substitution of the wild-type amino acid at X₃ by G or A,
4) a substitution of the wild-type amino acid at X₄ by A, and
5) a substitution of the wild-type amino acid at X₅ by S, A, N, or I.

6. The modified Nampt according to any one of claims 1 to 5, wherein the wild-type sequence of the modified Nampt has the following amino acid sequence (1) or (2):
(1) the amino acid sequence represented by any of SEQ ID NOs: 3, 5, 7, 9, 11, 13, 15, 17, 19, 21, and 23, and
(2) an amino acid sequence which has 90% or higher identity to the amino acid sequence represented by any of SEQ ID NOs: 3, 5, 7, 9, 11, 13, 15, 17, 19, 21, and 23 and encodes a protein having Nampt activity.

7. The modified Nampt according to any one of claims 1 to 6, wherein the modified Nampt is derived from the genus *Haemophilus,* the genus *Meiothermus,* the genus *Xanthomonas,* the genus *Sphingopyxis,* the genus *Sphingopyxis,* the genus *Chitinophaga,* the genus *Burkholderia,* the genus *Pedobacter,* the genus *Microbulbifer,* the genus *Labrenzia,* or the genus *Luteibacter.*

8. A method for producing nicotinamide mononucleotide by contacting phosphoribosyl pyrophosphate and nicotinamide with each other in the presence of a modified Nampt according to any one of claims 1 to 7.

9. The method according to claim 8, wherein the phosphoribosyl pyrophosphate is produced from ribose-5-phosphate in the presence of phosphoribosyl pyrophosphate synthetase.

10. The method according to claim 9, wherein the ribose-5-phosphate is produced from ribose in the presence of ribokinase.
